(19) Europäisches Patentamt
European Patent Office
Office européen des brevets

(11) **EP 4 491 725 A1**

(12) **EUROPEAN PATENT APPLICATION**
published in accordance with Art. 153(4) EPC

(43) Date of publication:
**15.01.2025 Bulletin 2025/03**

(21) Application number: **23766934.6**

(22) Date of filing: **09.03.2023**

(51) International Patent Classification (IPC):
*C12N 15/09* (2006.01)          *C12Q 1/68* (2018.01)
*C12Q 1/6869* (2018.01)          *C12Q 1/6888* (2018.01)

(52) Cooperative Patent Classification (CPC):
**C12N 15/09; C12Q 1/68; C12Q 1/6851;
C12Q 1/6869; C12Q 1/6888**

(86) International application number:
**PCT/JP2023/009099**

(87) International publication number:
**WO 2023/171758 (14.09.2023 Gazette 2023/37)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **09.03.2022 JP 2022036661**

(71) Applicant: **Kao Corporation
Tokyo 103-8210 (JP)**

(72) Inventors:
• **INOUE, Yasuaki
Haga-gun, Tochigi 321-3497 (JP)**
• **MIYATA, Kaede
Haga-gun, Tochigi 321-3497 (JP)**
• **HONDA, Hiroshi
Haga-gun, Tochigi 321-3497 (JP)**

(74) Representative: **Vossius & Partner
Patentanwälte Rechtsanwälte mbB
Siebertstrasse 3
81675 München (DE)**

Remarks:
The complete document including Reference
Table(s) and the Sequence Listing(s) can be
downloaded from the EPO website

(54) **FISH ECOLOGICAL SURVEY METHOD IN FRESHWATER ENVIRONMENT**

(57)     Reducing false positives in ecological surveys for a freshwater environment. A method for estimating a state of contamination of a freshwater environment with a fish nucleic acid derived from outside of the freshwater environment, comprising using on amounts of nucleic acids of environmental DNA and environmental RNA contained in domestic wastewater or influent of a wastewater treatment plant and effluent of the wastewater treatment plant.

**Description**

Field of the Invention

The present invention relates to an ecological survey method for fish in a freshwater environment.

Background of the Invention

Freshwater ecosystems provide resources and services important for humans. However, recently, the sustainability of freshwater ecosystems has been threatened by pollution sources such as domestic wastewater, industrial wastewater and agricultural water produced by human activities and flowing into water environments such as rivers and lakes.

To protect the freshwater ecosystem, it is indispensable to globally monitor the ecosystem. However, conventional ecological survey methods such as a method of directly capturing individual organisms by, e.g., fishing and traps, and a method depending on the observation of individuals by, e.g., checking eyes and shooting with a camera have problems in view of cost, completeness and reproducibility. One of the approaches for overcoming the problems of conventional ecological survey methods is metabarcoding analysis of environmental DNA (eDNA) (Non Patent Literatures 1 to 3). It is considered that the eDNA metabarcoding analysis comprehensively analyzes eDNAs discharged from individual aquatic organisms into a water environment to estimate biological species present in the water environment and the amount of the biological species. However, eDNA is stable and degrades slowly in the environment, and consequently has the potential to give a false positive. This is recognized as one of the major problems in applying eDNA metabarcoding analysis to ecological surveys (Non Patent Literatures 4 and 5).

As a method that can reduce false positives in the eDNA analysis, attention has been paid to metabarcoding analysis using environmental RNA (eRNA). Non Patent Literature 6 reports that fish eRNA is abundantly present in rivers; false positives are greatly reduced in an ecological survey by eRNA metabarcoding analysis, compared to surveys using eDNA; biological groups existing in survey sites were correctly detected by eRNA whereas saltwater and brackish water fish not existing in survey sites are detected by eDNA, i.e., false positives were generated; and most of all the saltwater and brackish water fish identified by the eDNA metabarcoding analysis were edible fish. Non Patent Literatures 7 and 8 disclose that, a large number of reads and diversity of reads detected by eDNA analysis for environmental samples reflect large amounts and diversity of biological species detected from the samples.

Non Patent Literature 1: Biol. Conserv., 2015, 183: 4-18
Non Patent Literature 2: Trends Ecol. Evol., 2014, 29 (6): 358-367
Non Patent Literature 3: Mol. Ecol., 2017, 26 (21): 5872-5895
Non Patent Literature 4: Annu. Rev. Ecol. Evol. Syst., 2018, 49 (1): 209-230
Non Patent Literature 5: Mol. Ecol. Resour., 2016, 16 (3): 604-607
Non Patent Literature 6: Ecol. Indic., 2021, 128, 107796
Non Patent Literature 7: Pros One, 2012, 7 (4): e35868
Non Patent Literature 8: Environmental DNA, 2021, 3: 105-120

Summary of Invention

In an embodiment, the present invention provides a method for estimating a state of contamination of a freshwater environment with fish nucleic acids derived from outside of the freshwater environment, comprising using amounts of nucleic acids of environmental DNAs and environmental RNAs, contained in domestic wastewater or an influent of a wastewater treatment plant and an effluent of a wastewater treatment plant.

In another embodiment, the present invention provides an ecological survey method using the above method for fishes in a freshwater environment.

Brief Description of Drawings

[FIG. 1] FIG. 1 shows the overview of a procedure for estimating the amount of a nucleic acid derived from false-positive fish species in a freshwater environment.
[FIG. 2] FIG. 2 shows quantitative results of eDNA and eRNA derived from fish and detected in domestic wastewater (a) and effluent (b).

[FIG. 3] FIG. 3 shows fish species detected by fish community analysis in domestic wastewater and effluent.

[FIG. 4] FIG. 4 shows the relationships between the number of eDNA reads and the number of eRNA reads from domestic wastewater and effluent. (a) Correlation between the number of eDNA reads and the number of eRNA reads derived from fish species detected in domestic wastewater, (b) the number of fish species detected from eDNA and eRNA in domestic wastewater, (c) Correlation of the number of eDNA reads and the number of eRNA reads derived from fish species detected in effluent, and (d) the number of fish species detected from eDNA and eRNA in effluent.

[FIG. 5] FIG. 5 shows decomposition behaviors of eDNA and eRNA derived from a test-water tank at 4 to 34°C.

[FIG. 6] FIG. 6 shows the contamination state of the nation's rivers with foreign nucleic acids (left: foreign eDNA, right: foreign eRNA).

[FIG. 7] FIG. 7 shows the contamination state of the Tone River with foreign nucleic acids (left: foreign eDNA, right: foreign eRNA).

[FIG. 8] FIG. 8 shows the contamination state of the Naka River with foreign nucleic acids (left: foreign eDNA, right: foreign eRNA).

Detailed Description of the Invention

From the reports of Non Patent Literature 6, it is considered that nucleic acids derived from edible fish discarded from, e.g., households, may have the possibility to produce false positives (false detection of fish species actually not present in a survey site) in an ecological survey by eDNA/eRNA metabarcoding analysis. The present inventors considered the possibility that nucleic acids derived from edible fish discarded from, e.g., households, are contained in domestic wastewater and effluent from wastewater treatment plants and produce false positives. In most cases, domestic wastewater is treated in wastewater treatment plants but sometimes directly discharged into rivers in the areas having a low wastewater treatment plant coverage rate per population.

For accurate ecological surveys for freshwater environments such as rivers using eDNA/eRNA metabarcoding analysis, it is important to estimate false positives and take a means for reducing the effects thereof.

As shown in Examples (later described), the present inventors conducted quantitative analyses of eDNA and eRNA contained in domestic wastewater and effluent from wastewater treatment plants and fish community analyses. As a result, they found that false positives are produced by nucleic acids derived from edible fish contained in domestic wastewater, and the like; and further found that nucleic acids causing false positives are contained in not only domestic wastewater but also the effluent from wastewater treatment plants (Experiment 1 described later). Furthermore, the present inventors found that, in the domestic wastewater not subjected to a wastewater treatment plant, the amount of eDNA is significantly larger than the amount of eRNA and thus eDNA can be a main cause of false positives in the areas having a low wastewater treatment plant coverage rate per population (Experiment 1). Furthermore, the present inventors confirmed, in eDNA and RNA stability tests, the conditions where the decay rate of eRNA becomes higher than that of eDNA (Experiment 2 described later).

From the above findings, in a comprehensive analysis of nucleic acids (for example, metabarcoding analysis) for the ecological survey of fish in freshwater environments, it is expected that the ecology of fish in freshwater environments can be more accurately evaluated by considering the effect of contamination of fish eDNA or eRNA derived from the external environment such as domestic wastewater. Furthermore, it is expected that ecological surveys by eRNA analysis can provide results with fewer false positives compared to eDNA in areas with low coverage of wastewater treatment plants.

In conventional ecological surveys of freshwater environment using a comprehensive analysis of nucleic acids, some measures have been taken to reduce false positives, such as selecting survey sites that are thought to be less affected by nucleic acids introduced from outside, but no attempt has been made to reduce false positives by estimating the amount of nucleic acid contaminants from outside or correcting/evaluating survey results based on the estimated results.

For the fish ecological surveys based on a comprehensive analysis of nucleic acids in freshwater environments, the present inventors developed a prediction model for estimating the contamination of fish eDNA or eRNA derived from outside the freshwater environment (hereafter also referred to as foreign eDNA or eRNA, which can cause false positives) in the freshwater environment to be surveyed. By reflecting the estimated effect on actual analysis results (for example, excluding the effect), survey results with reduced possibility of false positives can be obtained. More specifically, the amount or probability of a foreign eDNA or eRNA contaminant at survey sites is estimated by the prediction model. The amount of contaminants estimated is subtracted from the amount of eDNA or eRNA actually measured to reduce false positives. Alternatively, using the amount or probability of contaminants estimated as a standard, it is possible to evaluate whether the fish species detected is a false positive or not, based on actual measurement values. Alternatively, by predicting the risks of contamination separately with foreign eDNA and foreign eRNA at a survey site by using the prediction model, it is possible to determine which one of the measurements of eDNA and eRNA is suitable for an ecological survey at the survey site.

According to the present invention, it is possible, in ecological surveys of freshwater environments by comprehensive analysis of eDNAs or eRNAs, to estimate a false positive derived from nucleic acids from fish species actually not existing in the freshwater environment (for example, edible fish and the like contained in, e.g., domestic wastewater) and flowed into the survey sites and to reduce the effect of the nucleic acids.

In an embodiment, the present invention provides a method for estimating a state of contamination of a freshwater environment with fish eDNAs or eRNAs (foreign eDNAs or eRNAs) derived from outside of the freshwater environment. For example, in this method, the amounts (or concentrations) of foreign eDNAs or eRNAs or probability of contamination thereof are estimated at an arbitrary survey site in the freshwater environment. The estimated results can be used for obtaining more accurate results (e.g., exclusion of a false positive, evaluation of a false-positive possibility) of an ecological survey for fishes in a freshwater environment using comprehensive analysis of nucleic acids.

An embodiment of the present invention is directed to a method for estimating a state of contamination of a freshwater environment with fish nucleic acids derived from outside of the freshwater environment, using amounts of nucleic acids of eDNAs and eRNAs contained in domestic wastewater or an influent of a wastewater treatment plant and an effluent of the wastewater treatment plant. For example, the method includes estimating the state of contamination of the freshwater environment at a given site with the fish nucleic acids derived from outside, by using information on a site of contamination of the freshwater environment with the fish nucleic acids derived from outside and a decay rate constant or a half-life of the fish nucleic acids. Furthermore, the method includes, for example, estimating a species composition of fishes which derive the fish nucleic acids derived from outside of the freshwater environment, that is contaminated into the freshwater environment. Furthermore, for example, the method includes estimating the state of contamination of the freshwater environment at a given site with the fish nucleic acids derived from outside, by using the difference in an amount and composition of the nucleic acids contained in the domestic wastewater or an influent of a wastewater treatment plant and an effluent of a wastewater treatment plant, a wastewater treatment plant coverage and population at a site of contamination of the freshwater environment with the fish nucleic acids derived from outside.

Accordingly, an embodiment of the present invention relates to an ecological survey method for fishes in a freshwater environment employing comprehensive analysis of nucleic acids using an estimated value obtained by a method for estimating the state of contamination with the foreign eDNA or eRNA. In an embodiment, the ecological survey method is a method for detecting fish species existing in a freshwater environment. In another embodiment, the ecological survey method is a method for quantifying fish species existing in a freshwater environment.

In the embodiment, it is possible to correct the results of the ecological survey based on the estimated value obtained by the estimation method or to evaluate the accuracy of the results of the ecological survey. For example, the estimated values are subtracted from the results of comprehensive analysis of nucleic acids contained in an environmental sample taken at a survey site or the results are compared with the estimated values or the results are evaluated based on the estimated values. In this manner, detection or quantitative results of fish species are corrected to improve the accuracy of the ecological survey (for example, reduce false positives) and evaluate the accuracy of the ecological survey (for example, the presence of absence of a false positive).

In a representative embodiment, the present invention relates to a method for estimating false positives to reduce its effect in an ecological survey for a freshwater environment by comprehensive analysis of nucleic acids using the estimated value obtained by the method for estimating the state of contamination with the foreign eDNA or eRNA. In the present invention, it is possible to reduce the effect of a false positive on the analysis results by estimating the amount of foreign eDNA or eRNA at an ecological survey site, subtracting the quantitative value estimated from the quantitative value of a nucleic acid derived from a fish species actually obtained by an analysis. Furthermore, in the present invention, the amount of foreign eDNA or eRNA is estimated and whether a fish species is a false positive or not can be evaluated based on the quantitative value estimated.

In the specification, the "freshwater environment" refers to a water area such as a river, a lake, a pond and a swamp except a seawater area or a brackish water area. Examples of the freshwater environment include water and the bottom of water (for example, river bottom). The freshwater environment to be targeted by the present invention is preferably a river.

In the specification, the "environmental DNA (eDNA)" and "environmental RNA (eRNA)" refer to DNA and RNA, respectively which are contained in an environmental sample such as water, mud, bare rock and soil. Preferably, the environmental sample to be used for an ecological survey for a freshwater environment in the present invention is water or mud in the freshwater environment.

In the specification, the "false positive" in the ecological survey for a freshwater environment by global analysis of nucleic acids refers to false detection of a fish species not actually existing in the freshwater environment of a survey site or sometimes refers to a fish species falsely detected. The fish species falsely detected is sometimes referred to as a "false positive species".

In the specification, "foreign eDNA" and "foreign eRNA" (collectively referred to as "foreign nucleic acids"), refer to fish eDNA and eRNA derived from outside (for example, domestic wastewater, effluent from wastewater treatment plants) of the freshwater environment in an ecological survey for a freshwater environment by comprehensive analysis of nucleic acids. Foreign eDNA or eRNA, when it flows into a freshwater environment, produces a false positive in the ecological survey for the freshwater environment.

In the specification, the "false positive source" refers to a nucleic acid that may produce a false positive. The nucleic acid is preferably at least one selected from the group consisting of eDNA and eRNA, and more preferably eRNA. The false positive source is representatively eDNA or eRNA derived from a fish species having a possibility of a false positive. Accordingly, the false positive source may be included in the foreign nucleic acid. Examples of fish species having a possibility of a false positive include fish species that should not exist in the freshwater environment to be surveyed, such as fish species primarily existing in seawater or brackish water, freshwater fish and edible fish that should not virtually exist in the freshwater environment. Freshwater fish existing in the water area of the water environment, if it is caught, used as food and discarded in domestic wastewater, may produce a false positive.

In the specification, "domestic wastewater" refers to water, which is generated along with normal human life such as cooking and laundry and discharged. In the areas having a high wastewater treatment plant coverage rate per population, domestic wastewater is transported to a wastewater treatment plant, purified and discharged as an effluent into a freshwater environment such as a river. In contrast, in the areas having a low wastewater treatment plant coverage rate per population, domestic wastewater is directly discharged into a freshwater environment not via a wastewater treatment plant (site discharge). As shown in Experiment 1 described later, it was found that domestic wastewater contains foreign eDNA and foreign eRNA having a possibility of serving as a false positive source, and that the content of eDNA is significantly larger than eRNA. Accordingly, in the areas having a low wastewater treatment plant coverage rate per population, it is suggested that contamination with foreign eDNA may be more serious than with foreign eRNA.

The wastewater treatment plant refers to a facility for purifying domestic wastewater and is generally constituted of a settling tank, a primary sedimentation tank, an aeration tank (reaction tank), a final sedimentation tank and disinfection equipment. Foreign eDNA and foreign eRNA in domestic wastewater are greatly reduced by way of a wastewater treatment plant.

In the specification, the "influent of a wastewater treatment plant" (or influent) refers to the domestic wastewater flowing into a wastewater treatment plant including a mixture of domestic wastewater transported from a plurality of households. In the specification, the "effluent of the wastewater treatment plant" (or effluent) refers to water treated in the wastewater treatment plant and discharged in a freshwater environment. As shown in Experiment 1 described later, the effluent contains foreign eDNA and foreign eRNA that may be a false positive source, in a smaller amount than in domestic wastewater. For this reason, in urban rivers containing the effluent from the treatment plant in a large volume ratio to river water, it is indicated that a wide variety of false positive species are always detected.

In the specification, the "point source" and "non-point source" both refer to a site (hereinafter also referred to as a "site of contamination") at which water discharge containing foreign nucleic acids flows in a freshwater environment. When the site of contamination is specified, the site is referred to as a "point source". More specifically, in the case where a site of contamination at which water discharge flows into a freshwater environment can be specified, such as a port of effluent from a wastewater treatment plant and a discharge port of a single house with no wastewater treatment plant or no surrounding settlements, a site where water discharge assembled from several houses is discharged, the site of contamination is called a point source. In contrast, in the case where a site of contamination is not specified, the site of contamination is called a "non-point source". For example, in the areas having no sewage lines, a site where water discharge flows into a freshwater environment cannot be specified, the site of contamination is called a non-point source. In the areas having site discharge without passing through wastewater treatment plants, the site of contamination is highly possibly a non-point source. Accordingly, the discharge amounts of eDNA and eRNA are high, particularly the amount of eDNA is significantly larger than the amount of eRNA at the non-point source compared to a point source. Thus, eDNA has a high possibility of producing a false positive. Briefly, in the ecological survey for a freshwater environment by global analysis of nucleic acids based on the present invention, if the site of contamination is a non-point source, eRNA is more preferably analyzed in order to reduce false positives.

Now, the method of the present invention will be more specifically described.

1) Ecological survey for fish species by comprehensive analysis of nucleic acids

A nucleic acid contained in a freshwater environment is prepared in accordance with a routine method. The nucleic acid is preferably at least one selected from the group consisting of eDNA and eRNA, and more preferably eRNA. As a sample from a freshwater environment, water may be collected from surfaces or deep parts of, e.g., a river, a lake, a pond and a swamp, or mud may be collected from the bottom of them. DNA or RNA may be extracted from the obtained sample. DNA and RNA from a sample can be extracted in accordance with a routine method. For example, after a sample is filtered, if necessary, RNA or DNA contained in the filtrate is concentrated by, e.g., a nucleic acid adsorption filter and then extracted by a phenol/chloroform method, AGPC (acid guanidinium thiocyanate-phenol-chloroform extraction) method, or using, e.g., a commercially available DNA or RNA extraction reagent. DNA and RNA extracted may be directly used for analysis or may be stored (for example, cryopreserved under -20°C or -80°C) in accordance with a routine method.

Extracted eRNA derived from a sample may be subjected to various analyses directly in the form of RNA or converted into the form of DNA. Preferably, the eRNA derived from a sample is converted into cDNA by reverse transcription and then subjected to analysis. For the reverse transcription of RNA, a reverse transcriptase commonly used or a reverse transcription reagent can be used. Examples of the commercially available reverse transcription reagent include PrimeScript (registered trademark) Reverse Transcriptase series (Takara Bio Inc.), SuperScript (registered trademark) and Reverse Transcriptase series (Thermo Scientific).

Based on a comprehensive analysis of the eDNA or eRNA extracted, what fish species the eDNA or eRNA are derived from can be checked. In this manner, the fish species existing in the freshwater environment can be detected. Alternatively, based on comprehensive quantitative analysis of the eDNA or eRNA, the biomass amount of a fish species existing in the freshwater environment can be quantified.

Examples of the comprehensive analysis method for eDNA include DNA next-generation sequencing, DNA metabarcoding and DNA quantitative metabarcoding. The DNA metabarcoding is a method of selectively amplifying a marker DNA useful for identifying each fish species from eDNA extracted from a sample to prepare a library, sequencing the library, and comparing the determined sequence with the genomic sequence of each fish species. Examples of the marker DNA include DNA encoding marker RNA such as 12S rRNA and 16S rRNA useful for identification of species. The marker DNA can be selectively amplified by PCR using universal primers for fish, for example, MiFish (Miya et al., Royal Society Open Science, 2015, 2 (7): 150088, doi: 10.1098/rsos.150088). The genomic sequence of a fish species to be compared with the sequencing results can be obtained by a tool such as BLAST (blast.ncbi.nlm.nih.gov/Blast.cgi) .

Examples of the comprehensive analysis method for eRNA include RNA next-generation sequencing, RNA metabarcoding and RNA quantitative metabarcoding. In the eRNA comprehensive analysis, cDNA synthesized from the eRNA extracted is preferably used. The comprehensive analysis of eRNAs is performed in the same manner as in the comprehensive analysis of DNA mentioned above.

The comprehensive quantitative analysis of eDNA and eRNA can be performed with respect to a part of eDNA of fish species detected by the comprehensive analysis but preferably performed with respect to all fish species detected. Based on the quantitative analysis, the amount of eDNA or eRNA of each of the fish species detected by the comprehensive analysis is checked to quantify the biomass amounts of the fish species existing in the freshwater environment. The quantitative analysis may be performed based on the sequence reads obtained by, for example, the next-generation sequencing as mentioned above or sequencing in the metabarcoding. A large number of reads and diversity of reads reflect abundance in amount and diversity of species detected by eDNA or eRNA contained in a sample, that is, fish species existing in the freshwater environment (see, Non Patent Literatures 7 and 8). Accordingly, by computationally obtaining the correlation between the quantitative value (for example, the number of copies obtained by quantitative PCR and the number of sequence reads obtained by sequencing) of eDNA or eRNA derived from a predetermined fish species and the biomass amount of the fish species actually observed by a field survey, in advance, it is considered that the biomass amount of the fish species can be estimated from the amount of eDNA or eRNA.

2) Prediction model for estimation of the state of contamination with foreign nucleic acid

Into a freshwater environment such as a river, domestic wastewater or domestic wastewater or effluent from a wastewater treatment plant (hereinafter also referred to simply as "effluent") flows. In the specification, domestic wastewater and effluent are hereinafter collectively referred to as "water discharge". The water discharge contains nucleic acids derived from edible fish (eDNAs and eRNAs). The nucleic acids derived from edible fish in the water discharge may produce false positives in an ecological survey for fish species in a freshwater environment by comprehensive analysis of nucleic acids (Experiment 1 described later).

The present invention provides a prediction model for estimating the state of contamination (for example, the amount, concentration, and existence probability of a foreign nucleic acid) with a foreign nucleic acid in a freshwater environment. Now, the prediction model will be described below provide.

2.1) Exponential decay model

In an embodiment, the state of contamination with a foreign nucleic acid is estimated at an ecological survey site (hereinafter also referred to simply as a "survey site") by the prediction model of the present invention. In this model, in consideration of, e.g., the dynamics of eDNA and eRNA from a site of contamination to a survey site, more specifically, stability of the eDNA and eRNA in a freshwater environment, the dilution ratio in a freshwater environment and the time moving from a site of contamination to a survey site, the state of contamination (for example, the amount and concentration of a foreign nucleic acid) with a foreign nucleic acid at a survey site is estimated.

2.1.1) Overview of model

The amount of a foreign nucleic acid (for example, the number of reads or copies, the same will apply to the following description) at a site of contamination is calculated and the calculated value is defined as the initial amount $M_0$ of the foreign nucleic acid. Note that, the amount of a foreign nucleic acid can be calculated in the same manner as in the amount of nucleic acid in a freshwater environment. The time "t" until a foreign nucleic acid moves from a site of contamination to a survey site (passage time after discharge) is obtained. For example, the distance between the site of contamination and the survey site is obtained and divided by the flow velocity of a river to obtain the time "t".

A nucleic acid in a freshwater environment is assumed to be exponentially decomposed. The amount of a foreign nucleic acid at a survey site is calculated in accordance with the prediction model represented by the following formula (1):

$$M = M_0 e^{-kt} \quad \ldots \quad (1)$$

wherein

M: an estimated value of the amount of the foreign nucleic acid, at a survey site,
$M_0$: the amount of the foreign nucleic acid, at a site of contamination of a freshwater environment,
k: a decay rate constant, and
t: the time until the foreign nucleic acid reaches a survey site from the site of contamination.

Decay rate constant k is determined in advance in accordance with the procedure described later in 2.1.2.4).

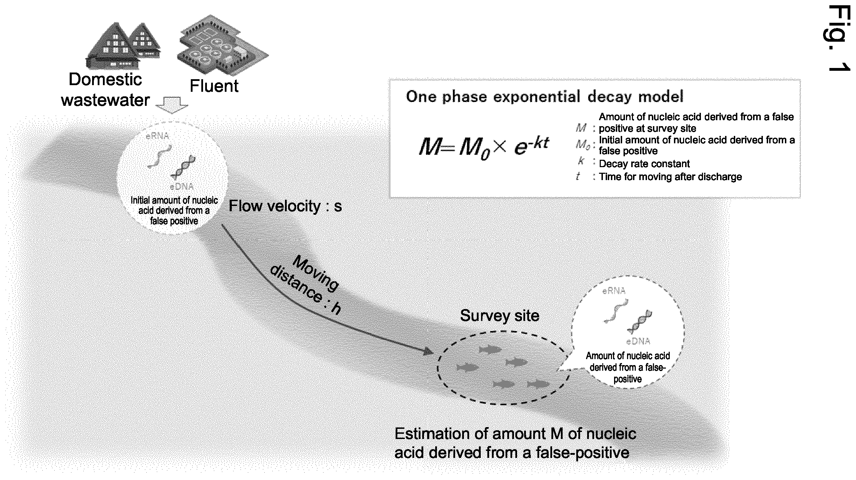

The value M representing the amount of the foreign nucleic acid at a survey site can be estimated in accordance with Formula (1) (FIG. 1). Note that if M to be estimated represents the amount of eDNA, $M_0$ represents the amount of eDNA, whereas, if M to be estimated represents the amount of eRNA, $M_0$ also represents the amount of eRNA.

When an estimated value M of the amount of a foreign nucleic acid at a survey site is subtracted from the actual measurement value of the nucleic acid at the survey site quantified by comprehensive analysis of nucleic acids explained in the above 1), the effect of a false positive on quantitative results of the global analysis can be reduced. Alternatively, a threshold of estimated value M is set with respect to various fish species, and fish species exhibiting M larger than the threshold can be regarded as a species having a high possibility of a false positive.

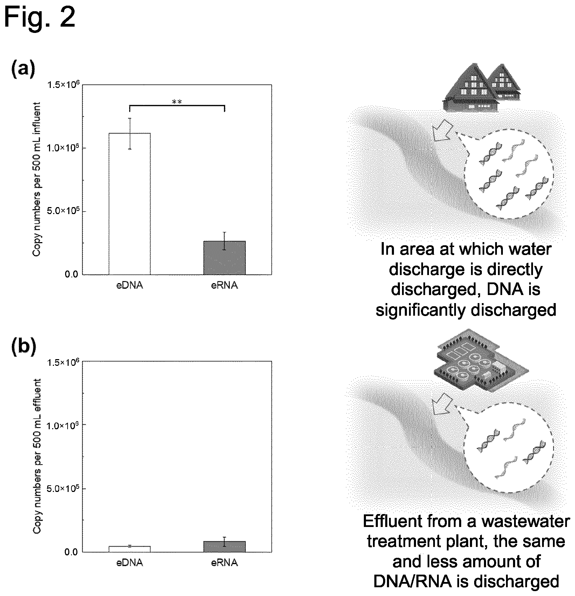

From the ratio of eDNA amount and eRNA amount in domestic wastewater shown in Experiment 1 described later), it was found that the amount of eDNA contained in the domestic wastewater is about 4 times as large as the amount of eRNA. In contrast, it was found that the eDNA amount contained in the effluent is the same as eRNA amount (FIG. 2). Accordingly, in the area having a low wastewater treatment plant coverage rate per population, the effect of foreign eDNA is estimated to be about 4 times as large as that of foreign eRNA. Accordingly, in consideration that the water discharge mainly flowing into a point source is domestic wastewater or effluent, employment of eDNA or eRNA as estimated value M and the magnitude of a threshold of estimated value M are preferably determined.

2.1.2) Construction of prediction model

Now, the prediction model represented by Formula (1) will be more specifically described. Note that, for the sake of simplicity of a prediction model and brevity of explanation, unless otherwise specified, it is provided that the site of contamination is a point source; the freshwater environment is a river; and the sample collected from the freshwater environment is water.

2.1.2.1) Initial amount $M_0$

The initial amount $M_0$ can be obtained by directly quantifying the nucleic acid amount at a point source of a freshwater environment. Alternatively, the initial amount $M_0$ can be calculated by dividing the quantitative value of a nucleic acid measured in water discharge itself (for example, domestic wastewater or effluent before discharge into a river) by the dilution ratio of the water discharge (with river water) at the point source.

The nucleic acid amount at the point source or of water discharge is obtained by quantitative analysis of a nucleic acid in a sample collected at the point source or of water discharge. Preferably, the nucleic acid is the same type as the nucleic acid quantified in comprehensive analysis explained in the above 1). More specifically, if the nucleic acid to be quantified in the procedure of the above 1) is eDNA, the amount of eDNA at the point source or of water discharge is obtained, whereas if the nucleic acid to be quantified in the procedure of the above 1) is eRNA, the amount of eRNA at the point source or of water discharge is obtained. The nucleic acid is more preferably, eRNA. The quantitative analysis of eDNA or eRNA in the point source or water discharge can be performed by, e.g., next-generation sequencing analysis, metabarcoding analysis, quantitative metabarcoding analysis, quantitative PCR, or digital PCR. The quantitative value is obtained, for example, as the number of sequencing reads obtained by metabarcoding analysis for DNA or RNA to be analyzed or the number of copies obtained by measurement by quantitative PCR. Preferably, eRNA is converted into cDNA, and thereafter, subjected to the quantitative analysis. The initial amount $M_0$ of a foreign nucleic acid is determined by the quantitative analysis. The water discharge dilution ratio can be obtained by the procedure later described.

2.1.2.2) Water discharge dilution ratio

The dilution ratio (discharge amount/river flow rate, also referred to as "water discharge dilution ratio") of water discharge with river water can be calculated by dividing the amount of water discharge to be discharged into a river by the flow rate of the river.

The flow rate of the river can be actually measured. For example, through the process of flow velocity measurement by, using e.g., a flow meter, flow rate calculation and preparation of a water-level flow rate curve, the flow rate can be calculated. It is desirable to use the flow rate near an ecological survey date but the statistically accumulated flow-rate data in the past can be used.

Alternatively, the flow rate of a river can be calculated from simulation. For example, the flow rate of a river is estimated from, e.g., an amount of rainfall, altitude and a land use rate, i.e., a method using distributed runoff model Hydro-BEAM (Hydrological river Basin Environment Evaluation Model) based on the regional geographic information. Hydro-BEAM is incorporated in AIST-SHANEL Ver.3.0 described later. Because of this, the flow rate of a river can be calculated using AIST-SHANEL Ver.3.0. For example, the flow rate of a river estimated by AIST-SHANEL in a 1-km mesh around a point source can be used.

The amount of water discharge released into a river can be estimated based on the statistical value of the amount of water used per person, regional population, and wastewater treatment plant coverage rate per population. When domestic wastewater and effluent are present together at a point source, the ratio of them can be obtained by calculating the ratio of domestic wastewater flowing into a wastewater treatment plant by AIST-SHANEL described later.

The ratio of domestic wastewater and effluent in water discharge can be estimated from the population and the wastewater treatment plant coverage rate per population. For example, the amounts of domestic wastewater and effluent can be estimated by AIST-SHANEL based on the information on population and the wastewater treatment plant coverage rate per population.

Furthermore, as a water-discharge dilution ratio, it is possible to use the statistical value of the water-discharge dilution ratio per river estimated by exposure analysis (river water concentration distribution analysis) of a chemical substance. The concentration distribution of a chemical substance in river water can be calculated by use of AIST-SHANEL. For example, the dilution ratio of site discharge in a 1-km mesh is 96 times, which was calculated by AIST-SHANEL and disclosed in "technical guidance of risk evaluation on priority assessment chemical substances in Act on the Evaluation of Chemical Substances and Regulation of Their Manufacture, etc. VI. exposure evaluation ... exposure scenario according to uses ... Ver.1.0 " (June, 2014, Ministry of Health, Labor and Welfare, Ministry of Economy, Trade and Industry, Ministry of the Environment) [www.meti.go.jp/policy/chemical_management/kasinhou/files /information/-ra/06_tech_guidance_vi_youtonioujita_v_1_0_1 40626.pdf] (hereinafter referred to as "guidance on the chemical substance regulation"). Accordingly, assuming that the dilution ratio of site discharge is 96 times, a method for predicting the amount of a foreign nucleic acid at a site of contamination is conceivable. Furthermore, the dilution ratio of the effluent of the wastewater treatment plant in a 1-km mesh calculated by AIST-SHANEL and disclosed in the guidance on the Act on the Evaluation of Chemical Substances and Regulation of Their Manufacture, etc. is 7 times. Accordingly, assuming that the dilution ratio of the effluent of the wastewater treatment plant is 7 times (corresponding to the least diluted case), a method for predicting the amount of a foreign nucleic acid at a site of contamination is conceivable.

Alternatively, the water-discharge dilution ratio can be calculated as the ratio of a concentration of a predetermined foreign nucleic acid in effluent and the concentration of the foreign nucleic acid in the river into which the nucleic acid is discharged. In this case, estimation and measurement of the amount of river water can be omitted.

2.1.2.3) Time, t

The distance between a point source and a survey site is obtained and divided by the flow rate of a river (flow velocity) to obtain the time t until a foreign nucleic acid discharged at the point source reaches the survey site from the point source.

2.1.2.4) Decay rate constant, k

A. Calculation from fish-breeding water

Specific procedures will be described in the following Experiment 2. Fish (fish species such as oryzias latipes generally used for experiments is acceptable) is raised in a water tank containing fish-breeding water (e.g., dechlorinated water or river water) for a certain period to allow eDNA and eRNA of the fish to elute in fish-breeding water. After the fish is removed, fish-breeding water is collected with time, and eDNA and eRNA in the collected fish-breeding water were quantified. For quantification, e.g., next-generation sequencing analysis, metabarcoding, quantitative metabarcoding, quantitative PCR, or quantification by digital PCR can be used.

The following items are obtained from the quantitative results.

$C_0$: Concentration of eDNA or eRNA derived from fish in fish-breeding water, at Time 0 (immediately after removal of fish from fish-breeding water).

C(m): Concentration of eDNA or eRNA derived from fish in fish-breeding water at Time m. The letter "m" represents the time required from Time 0 until eDNA and eRNA derived from fish in the fish-breeding water are quantified.

Decay rate constant, k can be calculated by fitting using the exponential decay model represented by the following formula (2):

$$C(m) = C_0 e^{-km} \quad \ldots \quad (2)$$

(C(m), $C_0$ and m are the same as defined above)

Since C(m) varies depending on the condition such as temperature or pH of fish-breeding water, decay rate constant k changes depending on the temperature or pH. The decay rate constant k in the condition such as temperature or pH corresponding to the condition such as temperature or pH of the river to be surveyed can be calculated in advance.

B. Calculation based on the initial amount $M_0$ and the amount of foreign nucleic acid at a survey site

The amount ($C_0$) of a foreign nucleic acid derived from a false-positive fish species (for example, saltwater species such as sardine and tuna) is measured at a point source. The amount (actual measurement value C(m)) of a nucleic acid derived from the false-positive fish species was measured at a survey site. Herein, m represents the time until a nucleic acid derived from a false-positive fish species moves from a point source to a survey site and can be obtained based on the distance from a site near the point source to the survey site and the flow velocity of the river.

Assuming that the amount of a nucleic acid reduces in accordance with the exponential decay model, C(m) = $C_0 e^{-km}$ of Formula (2) in the region from the point source to the survey site, the decay rate constant k can be calculated herein in accordance with the following formula (3):

$$k = \ln C_0 - \ln C(m) / t \quad \ldots \quad (3)$$

(C(m), $C_0$ and m are the same as defined in this paragraph)

In this method, "k", which is an intrinsic value to every fish species, can be estimated. Note that "k" intrinsic to a specific fish species estimated by this method can be applied to other types of fish species. Alternatively, the values "k" are individually estimated with respect to of a plurality of fish species and an average and median values of these can be used as a representative decay rate constant k.

2.1.3) Estimation of the amount of foreign nucleic acid using Formula (1), at survey site

The initial amount $M_0$, decay rate constant k obtained as mentioned above and time t are applied to Formula (1) to calculate the estimated value M of the amount of a nucleic acid derived from a false-positive fish species at a survey site, as shown later in Example 1.

2.2) Use of information on advection distance obtained by meta-analysis

The advection distance of a foreign nucleic acid flowing into a river can vary depending on fish species, water sampling situation and the flow rate of the river. Accordingly, it is conceivable to use a method for estimating the advection distance from the flow rate of a river based on the relationship between the flow rate of a river and nucleic acid advection distance, and a method for estimating the advection distance of a foreign nucleic acid from a point source by obtaining an average of the advection distances of eDNA or eRNA obtained in various researches. (Jo et al; DOI: 10.22541/au.163490255.53353112/v1, and Jo et al; DOI: 10.1111/1755-0998.13354). The state of contamination with a foreign nucleic acid at a survey site can be estimated based on the advection distance thus estimated. This method is a method obtained by further simplifying the exponential decay model. More specifically, when advection distance D at the flow rate of a survey site is reported, it can be qualitatively determined that a foreign nucleic acid discharged at a point source and a site near the point source can be detected at the site at a distance of D (meter) from the point source.

2.3) Prediction by exposure analysis model

Use of the advection distance information obtained by the exponential decay model or meta-analysis makes it possible to estimate the state of contamination with a foreign nucleic acid derived from a single point source arbitrarily chosen in a freshwater environment. However, there are a plurality of point sources in a river and the foreign nucleic acids discharged from the point sources may be distributed in an overlapped pattern. Further in the areas having a low wastewater treatment plant coverage rate per population, sometimes, non-point sources due to site discharge may present and the effect of contamination with a large amount of a foreign nucleic acid contained in the site discharge cannot be ignored. Furthermore, in the cases of point-source discharge from facilities in which sewage is not treated and a non-point source discharge, there is a high possibility that contamination with eDNA is more serious than eRNA. Accordingly, contamination with a large amount of a foreign nucleic acid due to a plurality of point source discharges and non-point source discharges, the volume ratio of eRNA and eDNA flowing into a river may impose a negative effect on the process for calculating an estimated value M. As a method for solving these problems, a method for using an exposure analysis model such as AIST-SHANEL described later is conceivable.

Accordingly, in another embodiment, the concentration distribution of a foreign nucleic acid in the entire river basin including a survey site is estimated by the prediction model of the present invention in consideration of a point source discharge and a non-point source discharge. In this model, it is possible to estimate the amount or concentration of a foreign nucleic acid existing in a freshwater environment, or the probability distribution of the presence of a foreign nucleic acid based on, for example, basin attributes per tertiary mesh (e.g., population, land use, industrial statistics, wastewater treatment plant coverage rate per population, altitude), weather information (e.g., amount of rainfall, temperature), physicochemical properties of chemical substances (vapor pressure, molecular weight, water solubility, organic carbon-normalized soil adsorption coefficient (Koc), half-life in river water, wastewater treatment plant removal rate). The distribution of a foreign nucleic acid can be estimated by the present model using national institute of Advanced Industrial Science and Technology-Standardized Hydrology-based Evaluation tool for chemical Exposure Load (AIST-SHANEL)" (Ver.3.0, available from the National Institute of the Advanced Industrial Science and Technology, the Research Institute of Science for Safety and Sustainability ([riss.aist.go.jp/shanel/]. AIST-SHANEL is software open to public, and used for, e.g., simulation of river pollution situation with chemical substances (Journal of Japan Society on Water Environment, 2012,35 (4): 65-72). Those skilled in the art can acquire AIST-SHANEL and apply it to the estimation of the distribution of a foreign nucleic acid by inputting parameters (e.g., the amounts of eDNA and eRNA contained in domestic wastewater and effluent, difference of eDNA and eRNA amounts contained in influent and effluent of a wastewater treatment plant, wastewater treatment plant removal rate of a foreign nucleic acid, and half-lives of eDNA and eRNA) obtained by a survey or inputting parameters obtained in Experiments 1 and 2 as shown in Example 2 as default values.

In this model, even if a site of contamination with a foreign nucleic acid cannot be clearly specified (for example, a case where the site of contamination is a non-point source, or a case where a point source and a non-point source are present together), it is possible to estimate the state of contamination with a foreign nucleic acid.

2.3.1) Overview of a model

AIST-SHANEL is a model for evaluating exposure of a chemical substance and countermeasures thereof in river basin in Japan and having functions to estimate the flow rates of rivers, the amounts of chemical substances discharged and the concentrations of chemical substances in river water. AIST-SHANEL can deal with weather information (e.g., amount of rainfall, temperature), in addition to data of basin attributes per tertiary mesh (e.g., population, land use, industrial statistics, wastewater treatment plant coverage rate per population, altitude). In the estimation of the concentrations of chemical substances in river water by AIST-SHANEL, a monthly average flow rate of a river per 1-km mesh is calculated based on the basin attributes per the tertiary mesh and weather information by distributed runoff model Hydro-BEAM (Hydrological river Basin Environment Assessment Model; incorporated into AIST-SHANEL Ver.3.0) based on regional geographic information. Furthermore, the discharge amounts of chemical substances are estimated based on basin attributes per tertiary mesh. Moreover, the concentrations of chemical substances in river water are calculated based on information of flow analysis and discharge amount. Examples of parameters of chemical substances relevant to the calculation of concentration include vapor pressure, molecular weight, water solubility, organic carbon-normalized soil adsorption coefficient (Koc), half-life in river water, and removal rate at wastewater treatment plants. As an example of parameters having an effect on estimation of chemical substance concentrations using a model, the load discharge coefficient of waterway bottom sediment (bed-load coefficient) is known (Journal of Japan society on Water Environment, 2018, Vol. 41, No. 5, page 129 to 139) .

AIST-SHANEL is a model for evaluating exposure of chemical substances in river basin and measures thereof in Japan and has an advantage in using estimation of the concentration distribution of a foreign nucleic acid.

First, in AIST-SHANEL, a monthly average flow rate of a river per 1-km mesh can be accurately obtained using the aforementioned Hydro-BEAM based on weather information (e.g., amount of rainfall, temperature) in addition to data of the basin attributes per the tertiary mesh (e.g., population, land use, wastewater treatment plant coverage rate per population, altitude). The flow rate of a river is useful for obtaining the water-discharge dilution ratio, as mentioned above. Furthermore, the amounts of foreign eDNA and eRNA in domestic wastewater flowing into a wastewater treatment plant can be estimated by multiplying the amount of water discharge estimated (surrounding population $\times$ use amount of water) to the amounts of foreign eDNA and foreign eRNA in water discharge, using information on a wastewater treatment plant and surrounding population.

Second, the locational information of wastewater treatment plants is incorporated into AIST-SHANEL and useful for analyzing the behavior of contaminants discharged at a point source from a wastewater treatment plant and advected. Thus, it is possible to estimate the state of contamination with a foreign nucleic acid in consideration of the overlapping concentration distribution of a foreign nucleic acid in river water derived from a plurality of point sources. More specifically, the state of contamination with a foreign nucleic acid at a predetermined location was estimated by the exponential decay model previously mentioned in the above 2.1), but in this model, the state of contamination with a foreign nucleic acid can be estimated over the entire river basin in consideration of the number of wastewater treatment plants actually present and locations thereof.

Third, in AIST-SHANEL, using information on basin attributes per the tertiary mesh (population, wastewater treatment plant coverage rate per population), the amount of water discharge at a non-point source (surrounding population $\times$ amount of water used $\times$ (1 - wastewater treatment plant coverage rate per population)) can be calculated. In a survey using eDNA and eRNA, a case where the state of contamination with a foreign nucleic acid derived from a non-point source was evaluated cannot be confirmed. Accordingly, this approach makes it possible to simulate the possibility of detection of a false positive species by discharge of domestic wastewater at a non-point source with respect to each of eDNA and eRNA. In the domestic wastewater not treated in wastewater treatment plants, the amount of eDNA is significantly larger than the amount of eRNA. Thus, in the areas having a low wastewater treatment plant coverage rate per population, contamination with eDNA may become severer. Depending on the estimated degree of contamination with eDNA and eRNA at a survey site, information regarding survey design as to which one of analyses for is useful can be obtained in advance.

Fourth, AIST-SHANEL has an advantage in that the concentration distribution of a nucleic acid in river water can be estimated in consideration of physicochemical properties of nucleic acids. Examples of parameters for a chemical substance include vapor pressure, molecular weight, water solubility, organic carbon-normalized soil adsorption coefficient (Koc), half-life in river water, and wastewater treatment plant removal rate. In particular, it is advantageous to estimate the distribution by disappearance in water and advection in consideration of the half-life in river water, as the difference between eDNA and eRNA.

As shown in Experiment 1 described later, it was found that the amount of eDNA in domestic wastewater is significantly higher than the amount of eRNA. The amount of eDNA derived from domestic wastewater was about 4 times as large as the amount of eRNA (FIG. 2). Thus, the amount of eDNA derived from a false-positive fish species and flowing into the freshwater environment through site discharge of domestic wastewater is estimated to be about 4 times as large as the amount of eRNA. Accordingly, also in other water areas and other wastewater treatment plants, the effects of point source discharge from neighborhood water through a wastewater treatment plant and non-point source from site discharge can be surveyed by measuring the ratio of eDNA and eRNA in domestic wastewater, influent and effluent. More specifically, in simulation by AIST-SHANEL, as shown in examples of false positive evaluation using a prediction model carried out in Example 2 described later, it is possible to arbitrarily set the initial concentrations of foreign eDNA and foreign eRNA such that the amount of eDNA is several times, as estimated, (for example, 4 times) as large as that of eRNA in domestic wastewater, or to calibrate various parameters (in the analysis using AIST-SHANEL, the load discharge coefficient of waterway bottom sediment (bed-load coefficient) and the like) involved in the estimation of nucleic acid concentration.

Furthermore, the present inventors found that the amount of a nucleic acid (eDNA or eRNA) discharged from any one of the wastewater treatment plants present in this country can be estimated based on the ratio of population. More specifically, the amount of a nucleic acid discharged from a wastewater treatment plant of interest can be predicted in accordance with the following formula.

The population to be treated in a wastewater treatment plant: nucleic acid amount in an effluent from the wastewater treatment plant = the population to be treated in a wastewater treatment plant of interest: nucleic acid amount in an effluent from the wastewater treatment plant of interest

Information on population to be treated in a given wastewater treatment plant is available from AIST-SHANEL. The nucleic acid amount in the effluent of a given wastewater treatment plant can be estimated by quantitative analysis for a nucleic acid in samples taken from water discharge as mentioned above or analysis using AIST-SHANEL, as mentioned above.

Furthermore, from the studies of the present inventors, the removal rate of eRNA in a wastewater treatment plant is calculated as about 68% and the removal rate of eDNA in a wastewater treatment plant is calculated as about 96%.

• Removal rate of eRNA in wastewater treatment plant = 1 - (the amount of eRNA derived from fish in effluent/the amount of eRNA derived from fish in influent) $\times$ 100 = 1 - (83955.6 (copies/500 mL effluent)/265779 (copies/500 mL influent)) $\times$ 100 = 68%

• Removal rate of eDNA in wastewater treatment plant = 1 - (the amount of eDNA derived from fish in effluent/the amount of eDNA derived from fish in influent) $\times$ 100 = 1 - (47111.1 (copies/500 mL effluent)/1114074 (copies/500 mL influent)) $\times$ 100 = 96%

Using these removal rates of eDNA and eRNA in wastewater treatment plants makes it possible to realize a spatial-temporal concentration distribution analysis in consideration of the amounts of eDNA and eRNA discharged from all point sources of nationwide rivers.

For example, by inputting the amounts of eDNA and eRNA in effluent from a neighborhood wastewater treatment plant and in domestic wastewater from site discharge, at a given site, the removal rate of eDNA or eRNA in the wastewater treatment plant as defined above, and half-life in water, to AIST-SHANEL, the estimated value M of a foreign nucleic acid amount at each site can be calculated.

The type of fish most frequently eaten differs depending on the area, if quantitative analysis of eDNA or eRNA at a point source in each area is performed, it is possible to realize more precise prediction of the amount of contamination with a false positive source per fish species. In contrast, also in water discharge at a non-point source, it is possible to realize estimation of concentration distribution assigned as a non-point source in consideration of site discharge by AIST-SHANEL analysis. The concentration distribution estimated can be enhanced in accuracy by taking calibration and a scale factor into consideration.

2.4) Other prediction models

A prediction model can be constructed based on metadata obtained by using various modeling methods conventionally employed in eDNA dynamics studies in addition to the methods mentioned above. Representative cases will be outlined.

2.4.1) Bayesian hierarchical modeling

A model is assumed to have a plurality of boxes connected. Flow-down of eDNA or eRNA, attenuation and observation thereof are expressed by Bayesian hierarchical modeling. The distribution of a false positive species and the detection probability thereof at a survey site are conceivably calculated by the modeling of prediction. Such modeling for eDNA has been already reported (the eDNA Society, 2021, the eDNA Society, 4th conference, November 20 and 21, 2021, presentation number PP053, Aoba Itoh, et al.). According to the modeling, a change in the amount of a nucleic acid (eDNA or eRNA) at a survey site can be defined as (amount of nucleic acid flowing down from upstream) + (amount of nucleic acid flowing into a survey point) - (amount of nucleic acid flowing to downstream) - (amount of nucleic acid decomposed at a survey site). Note that, the amount of a nucleic acid can be defined as a product obtained by multiplying the analysis results of eDNA or eRNA (reads/m$^3$ if metabarcoding analysis) by the flow rate (m$^3$) of a river. Note that, if AIST-SHANEL is used, the discharge amount of a point source or a non-point source can be input as the amount of nucleic acid flowing into a survey site and the flow rate of a river can be analyzed as mentioned above. Besides, observation interval and decay rate are used as parameters and the Bayesian hierarchical modeling is constructed using prior distribution. Next, parameters are input in the modeling. A method for simultaneously estimating biological distribution and the decay rate of a nucleic acid from observation data at a variety and plurality of sites (results of metabarcoding analysis of eDNA or eRNA) and the basic physical data of a river (e.g., distance from a river mouth to a water sampling point) is conceivable. The decay rate of a nucleic acid can be used as reference information to decay rate constant k; and the biological distribution information is effectively used for estimating a false positive species detectable by metabarcoding analysis.

2.4.2) Site occupancy modeling

It can be considered to employ a method using hierarchical modeling in consideration of a detection error of species based on results of a survey conducted based on a plurality of eDNA or eRNA samples measured at a plurality of sites. In consideration of a site occupancy probability by a species, probability of capturing a sequence per species and relative priority of a sequence, modeling is applied by Bayesian estimation using MCMC (Methods in Ecology and Evolution, 2022, 13 (1): 183-193). Analysis can be made using R package occumb (rdrr.io/github/fukayak/occumb/f/). As parameters, metabarcoding analysis results, the number of survey sites, and the number of water-sampling repeats are input. Outputs are site occupancy probability per species or per nucleic acid, probability of capturing a sequence per species, *ex ante* probability of relative priority of a sequence and *ex post* sample based on the probability.

In order to apply this method to analysis for a false positive species, the type of nucleic acid (eDNA or eRNA) to be subjected to metabarcoding analysis and information on the possibility or impossibility of a false positive species, which is obtained in advance by performing eDNA and eRNA analysis at a point source near a survey water area are input as covariates. In this way, the effects of these covariates on site occupancy probability per species or per nucleic acid, the probability of capturing a sequence, and relative priority of the sequence, can be analyzed. When information on the possibility or impossibility of a false positive species is not added as a covariate, false positive species alone is extracted from the results of metabarcoding analysis and subjected to R package occumb. In this way, the detection probability of a false positive species in a survey water-area can be precisely predicted. Furthermore, if the number of repeats, the number of survey sites, and an *ex-post* sample per type of nucleic acid to be subjected to metabarcoding analysis are obtained by the Bayesian decision analysis, it is possible to previously search the number of sites for sampling water for maximizing the detection probability of non-false positive species (the number of false negatives is reduced) and minimizing the detection probability of false positive species, the number of repeats for water sampling, and the type of nucleic acid to be subjected to metabarcoding.

As mentioned in the foregoing, since the detection probability of the nucleic acid derived from a false positive species per site and species can be found by applying this method, the method is thought to be useful for consideration of false positive species. In the aforementioned analysis using AIST-SHANEL, a spatial-temporal concentration of false-positive nucleic acids derived from point source and non-point source discharge can be analyzed over entire river basins. In contrast, this method cannot estimate the concentration and is only applicable to a water area analyzed by metabarcoding analysis. However, this method can evaluate a false positive risk from various detection probabilities. For the reason, the present method is useful for optimizing analysis conditions such as water sampling conditions and the number of sites.

2.4.3) Ecosystem model

Examples of river ecosystem models so far known include one-dimensional substance dynamic simulation (constituted of River flow model, Heat balance model, and Material Transport model), and two-dimensional substance dynamic simulation (using Spatial distribution prediction model and biological growth model in addition to one-dimensional calculation) (see, collection of papers of the Japan Society of Civil Engineers B1 (hydraulic engineering), 2018, 74 (5): I_409-I_414). In the model of the present invention, simulation is performed based on information such as the amount of river water and temperature. The advection distance can be estimated by the model in consideration of advection, diffusion and sedimentation process thereof, and whether a false positive species can be detected or not at a measurement site can be predicted in advance. Examples of parameters relevant to sedimentation of a nucleic acid include sedimentation velocity, particle size, particle density, fluid density, gravitational acceleration, and fluid viscosity. Examples of parameters for one-dimensional substance dynamic simulation include the cross-sectional area of flowing water, flow rate, riverbed height, gravitational acceleration, water depth, energy gradient, water intake per unit length in a flow direction, nucleic-acid concentration of each cross section, sedimentation velocity, dispersion coefficient, and water level. Calculation is carried out using these parameters. If dynamic simulation is performed based on the average particle size and amount of a false-positive nucleic acid, it is possible to obtain more highly accurate information than the estimation using a half-life and a flow rate, alone, with respect to the advection distance of a false-positive nucleic acid derived from a point source.

2.5) Reduction of false positives using estimated value M

The estimated value M of a foreign nucleic-acid amount obtained by the prediction model mentioned above is subtracted from the quantitative result actually measured to reduce the effect of false positives on the quantitative results. More specifically, from the quantitative value of a nucleic acid derived from a fish having a possibility of a false positive at a survey site and obtained by comprehensive analysis of nucleic acids explained in the above 1), the estimated value M of a fish species having a possibility of a false positive is subtracted, to reduce the effect of a false positive on the quantitative value. When the estimated value M is an amount of eDNA, the actual measurement value is an amount of eDNA, whereas when the estimated value M is an amount of eRNA, the actual measurement value is an amount of eRNA. When the estimated value M is the number of reads obtained by metabarcoding analysis, the actual measurement value is the number of reads obtained by metabarcoding analysis. When the estimated value M is the number of copies obtained by, e.g., quantitative PCR or digital PCR, the actual measurement value is the number of copies obtained by, e.g., quantitative PCR or digital PCR.

2.6) Estimation of false-positive fish species

A false positive species that can be potentially detected at a survey site, can be estimated by a qualitative analysis based on the amount of eDNA or eRNA detected at a point source. Assuming that eDNA or eRNA obtained by metabarcoding analysis at a point source moves by the advection distance obtained by, e.g., the meta-analysis or exponential decay model as mentioned above, the possibility that a fish species detected at a survey site is a false positive can be simply evaluated. In other words, in a case where the species detected by the analysis of eDNA or eRNA at a survey site is detected by eDNA or eRNA in water discharge within an estimated advection distance, it is considered that the species is possibly a false positive. Further, when the amount of eDNA or eRNA at a point source is not quantified, it is considered that analysis results of the influent and effluent of a neighborhood wastewater treatment plant and domestic wastewater can be used in place. As described later in Example 2, the present inventors demonstrated that the composition of fish species from which a false positive source in water discharge is derived, significantly differs before and after the treatment step of a wastewater treatment plant.

As shown in Example 2, the areas contaminated with eDNA or eRNA from domestic wastewater are mapped. As a result, it is considered that false-positive fish species detected include fish species from which a false-positive source in effluent is derived in urban areas having a large effluent ratio, whereas false-positive fish species include fish species from which a false positive source in domestic wastewater is derived in the areas around which no neighbor wastewater treatment plants are present and which is considered to be contaminated due to site discharge from a non-point source,. In AIST-SHANEL, point source discharge and non-point source discharge can be analyzed separately. Because of this, based on evaluation of the contamination amount of a false positive source from each of a point source and a non-point source, a false positive flowing into a survey site can be estimated. Particularly, the estimation of a false positive derived from a non-point source by this method is performed by eDNA/eRNA metabarcoding analysis in domestic wastewater or influent in consideration of food habits in the surrounding water area. In this respect, it is considered that the method has high novelty.

The degrees of contamination with eDNA and eRNA are evaluated by use of AIST-SHANEL in the entire river basin to estimate a site where a fish species can be detected and more accurately evaluate the possibility that the fish species detected is a false positive. In the analysis by AIST-SHANEL, it is possible to obtain information on the relative contamination ratio of eDNA and eRNA based on the consideration that the contamination amounts of eDNA and eRNA differ depending on whether the water discharge is via a treatment plant or site discharge. At a site where the ratio of eDNA and eRNA of a false positive species flowing into a survey site is predicted to deviate significantly from 1:1, metabarcoding analysis of both eDNA and eRNA are employed. Whereby, trustworthy false-positive estimation can be made. For example, when the ratio of eDNA and eRNA at a survey site is predicted as 5: 1, the fish species having a ratio of reads of eDNA and eRNA of 5: 1, which is obtained by metabarcoding analysis at the survey site, is determined to be possibly a false positive.

Alternatively, the threshold for determining a false positive is set per fish species in advance. If a value obtained by subtracting an estimated value M from the actual measurement value of a nucleic acid amount (for example, reads by quantitative metabarcoding analysis) for a certain fish species is larger than the threshold (for example, 10 reads, which is a standard determined as an error in sequencing), it is possible to estimate that the fish species exists in a freshwater environment (not a false-positive fish species). As the threshold, the detection limit concentration of a nucleic acid by a quantitative method can be employed.

Alternatively, samples prepared by stepwise dilution are subjected to the quantitative analysis using, e.g., quantitative PCR and digital PCR to determine the detection limit concentration of a nucleic acid in a sample. Depending on whether or not the estimated values M of various fish species exceed the detection limit concentration, it is determined that the fish species can be detected or not.

In the following embodiments of the present invention, substances, production methods, uses, methods and others will be further disclosed but the present invention is not limited to these embodiments.

<1> A method for estimating a state of contamination of a freshwater environment with fish nucleic acids derived from outside of the freshwater environment, comprising using amounts of nucleic acids of environmental DNAs and environmental RNAs contained in domestic wastewater or an influent of a wastewater treatment plant and an effluent of the wastewater treatment plant.

<2> The method according to <1>, preferably comprising quantifying the amounts of nucleic acids of environmental DNAs and environmental RNAs contained in the domestic wastewater or the influent of the wastewater treatment plant and the effluent of the wastewater treatment plant.

<3> The method according to <1> or <2>, preferably comprising estimating the state of contamination of the freshwater environment at a given site with the fish nucleic acids derived from outside, by using information on a site of contamination of the freshwater environment with the fish nucleic acids derived from outside and a decay rate constant or a half-life of the fish nucleic acids.

<4> The method according to <3>, wherein preferably the site of contamination is a site where the effluent of the wastewater treatment plant is discharged into the freshwater environment or a site where the domestic wastewater is discharged into the freshwater environment.

<5> The method according to any one of <1> to <4>, preferably comprising estimating a species composition of fishes which derive the fish nucleic acid derived from outside of the freshwater environment, that is contaminated into the freshwater environment.

<6> The method according to any one of <1> to <5>, preferably comprising, estimating the state of contamination of the freshwater environment at a given site with the fish nucleic acids derived from outside, using the difference in an amount and composition of the nucleic acids contained in the domestic wastewater or an influent of a wastewater treatment plant and an effluent of the wastewater treatment plant, a wastewater treatment plant coverage rate and a population at a site of contamination of the freshwater environment with the fish nucleic acid derived from outside.

<7> An ecological survey method for fishes in a freshwater environment using the method according to any one of <1> to <6>.

<8> The method according to <7>, preferably comprising:

comprehensively quantifying amounts of nucleic acids derived from fish species in a sample collected at a survey site in the freshwater environment;

calculating an estimated value M of an amount of a nucleic acid derived from a fish species having a possibility of a false positive in the sample based on the following formula (1):

$$M = M_0 e^{-kt} \quad \ldots \quad (1)$$

wherein

M represents an estimated value of an amount of the nucleic acid derived from the fish species having a possibility of a false positive, at the survey site,

$M_0$ represents an amount of the nucleic acid derived from the fish species having a possibility of a false positive, at a site of contamination where the nucleic acid contaminates into the freshwater environment,

k represents a decay rate constant, and

t represents the time until the nucleic acid derived from the fish species having a possibility of a false positive reaches the survey site from the site of contamination; and,

reducing false positives in results of the quantification based on the estimated value M.

<9> The method according to <8>, preferably comprising subtracting the estimated value M from a quantitative value of the nucleic acid derived from the fish species having a possibility of a false positive measured from the sample collected at the survey site, thereby reducing an effect of a false positive on the quantitative value.

<10> The method according to <9>, preferably comprising estimating that the fish species having a possibility of a false positive is determined as not a false positive when the value obtained by subtraction is a predetermined value or more.

<11> The method according to any one of <8> to <10>, preferably comprising estimating that a fish species is determined as a false positive when the estimated value M is a threshold value or more.

<12> The method according to any one of <8> to <11>, wherein preferably, the nucleic acid is at least one selected from the group consisting of environmental DNA and environmental RNA.

<13> The method according to any one of <8> to <12>, wherein preferably, the comprehensive quantification is quantification by a next-generation sequencing analysis, a metabarcoding analysis, or a quantitative metabarcoding analysis.

<14> The method according to any one of <1> to <13>, wherein preferably, the freshwater environment is a river.

Examples

The present invention will be more specifically described based on Examples but the present invention is not limited to these.

Experiment 1. Quantification of eDNA and eRNA derived from fish contained in domestic wastewater and effluent

1.1) Sampling of domestic wastewater and effluent

The domestic wastewater and effluent to be subjected to tests were collected from wastewater treatment plants in Tochigi Prefecture, Japan from July to November, 2020 (over 3 periods, 3 samples were taken per period, 9 samples in total). The water discharge samples collected were placed in an ice bath and transported to a laboratory. Since the water discharge samples contained a solid content, the samples were each subjected to suction filtration using a glass filter (pore

size 1.0 μm) to separate the solid content and a filtrate.

1.2) Nucleic acid extraction and cDNA synthesis

DNA and RNA were extracted separately from the resultant solid contents and filtrates. DNA extracts or RNA extracts obtained from the solid content and filtrate respectively are combined to obtain a DNA extraction liquid and an RNA extraction liquid. To describe more specifically, the solid content was removed from the filter and RNA and DNA were separately extracted using RNeasy PowerSoil Total RNA Kit and RNeasy PowerSoil DNA Elution Kit (Qiagen) in accordance with the recommended protocol. The filtrate was passed through Sterivex™ filter (pore size 0.45 μm: Millipore) to allow nucleic acids to adsorb thereon. To the Sterivex™ filter, an elution buffer (a mixture of PowerBead Solution (1.25 mL) and Solution SR1 (0.25 mL) (RNeasy PowerSoil Total RNA Kit, QIAGEN)) was added to elute nucleic acids in accordance with the method of Miya, et. al. (J Vis Exp, 2016, doi: 10.3791/54741). The eluate was transferred to a PowerBead Tube and PowerBead Solution (1.25 mL), Solution IRS (0.8 mL), and phenol/chloroform/isoamyl alcohol (3.5 mL) were added thereto, and stirred by a vortex for 15 minutes, to extract RNA and DNA separately in accordance with the recommended protocols of RNeasy PowerSoil Total RNA Kit and RNeasy PowerSoil DNA Elution Kit (Qiagen). RNA extracts and DNA extracts obtained from each of solid contents and filtrates of individual samples, are combined to obtain an RNA extraction liquid and a DNA extraction liquid per water discharge sample.
The RNA extraction liquid was subjected twice to the recommended protocol of rDNase Set (MACHEREY-NAGEL) and NucleoSpin RNA Clean-up XS (MACHEREY-NAGEL) to remove a residual genomic DNA and contaminants. The DNA extraction liquid was purified in accordance with the recommended protocol of NucleoSpin gDNA Clean-up XS (MACHEREY-NAGEL) to remove contaminants.
Using the obtained RNA extraction liquid as a template, cDNA was synthesized by using PrimeScript II 1st strand cDNA Synthesis Kit (Takara Bio Inc.) in accordance with the recommended protocol. In order to check the presence or absence of cross-contamination during DNA and RNA extraction and cDNA synthesis cDNA was synthesized using deionized water as a control in the same manner as above.

1.3) Quantification of eDNA and eRNA derived from fish in sample

1.3.1) Quantitative analysis

Using QX200 AutoDG Droplet Digital PCR System (Bio-Rad Laboratories), eDNA and eRNA derived from fish and contained in each water discharge sample were quantified. As the primers, fish universal primers, MiFish-U and - E33, targeting 12S rRNA gene, were used. A Droplet Digital (dd) PCR reaction solution was prepared by mixing 10 μM Forward and Reverse primers, QX200 ddPCR EvaGreen Supermix, and purified water. On a 96-well plate, template DNA (DNA extraction liquid or cDNA prepared in the above section 1.2)) and a ddPCR reaction solution were mixed, and thereafter, ddPCR reaction droplets were prepared by use of an Automated droplet generator. These were transferred to a PCR plate and PCR was performed by a C1000 Touch thermal cycler. In PCR, a first cycle of heat denaturation consisting of a reaction at 95°C for 5 minutes, a reaction at 94°C for 30 seconds and a reaction at 61°C for 1 minute was repeated 40 times and the enzyme was inactivated at 98°C for 10 minutes and thereafter, the plate was kept at 4°C. Thereafter, the amount of a nucleic acid was measured by QX200 Droplet Reader. On each ddPCR plate, a negative control (RNA/DNA free water) and a positive control (genomic DNA extracted from oryzias latipes) were set. The measurement results were analyzed by QuantaSoft Version 1.7.4 (Bio-Rad). The threshold for determining positive droplets was determined in accordance with the instruction of QuantaSoft. The uniformity of dispersion was determined by using an F test, uniform dispersibility was exhibited, and thus, a paired samples t-test was performed to determine whether or not the DNA amount and the RNA amount in domestic wastewater significantly differ. In the following Examples, all statistical analyses were performed using Origin Pro 2021b (OriginLab) and p value < 0.05 is regarded as statistically significant.

1.3.2) Results

As a result of the quantitative analysis, eRNA was detected in water discharge samples. This was a surprising result since it was already predicted that eRNA is not present in the water discharge samples in view of stability. In domestic wastewater, a larger amount of a nucleic acid was detected compared to the effluent. Furthermore, eDNA was statistically significantly detected in a larger amount compared to eRNA (FIG. 2 (a)). Accordingly, in the area where a sewerage system is not developed and domestic wastewater is directly discharged into a river, it is considered that a significantly larger amount of eDNA is discharged than eRNA. In contrast, the amount of nucleic acids in the effluent was greatly lower compared to domestic wastewater, and the amounts of eDNA and eRNA were equal. From this, it was shown that a wastewater treatment plant greatly contributes to a reduction of false positives (FIG. 2 (b)). However, even in the effluent, nucleic acids were not always completely removed and eDNA and eRNA derived from water discharge were found to be contained even in a low amount.

1.4) Survey of fish DNA and RNA contained in domestic wastewater and effluent (fish community analysis)

1.4.1) Construction of amplicon library and MiSeq sequencing

An amplicon library of 12S rRNA gene was prepared using fish universal primers, MiFish-U and -E33, by Two step tailed PCR method. In the library preparation procedure, 1st PCR was carried out in accordance with the recommended protocol of TaKaRa Ex Taq Hot Start Version (Takara Bio Inc.). As a template, the DNA extraction liquid or cDNA prepared in the above 1.2) was used and the concentrations of Forward and Reverse primers were set at 0.5 $\mu$M. PCR conditions were as follows: a first cycle of heat denaturation consisting of a reaction at 94°C for 2 minutes, a reaction at 94°C for 20 seconds, a reaction at 65°C for 15 seconds and a reaction at 72°C for 20 seconds was repeated 35 times, and the final cycle of extension was performed at 72°C for 5 minutes. Individual samples were subjected to the 1st PCR, 8 times, and then, the obtained PCR products of 8 batches were combined into one, which was subjected to the 2nd PCR. In the 2nd PCR, a 0.5 $\mu$M primer pair containing a MiSeq adapter sequence and an 8 bp index sequence were used to link to the ends of an amplicon. The conditions of the 2nd PCR were as follows: a first cycle of heat denaturation consisting of a reaction at 94°C for 2 minutes, a reaction at 94°C for 30 seconds, a reaction at 60°C for 30 seconds and a reaction at 72°C for 30 seconds was repeated 12 times and the final cycle of extension was performed at 72°C for 5 minutes. The obtained library was subjected to sequencing by Illumina MiSeq Reagent Kit v3 (600Cycle) for 2 $\times$ 300 bp PE (Illumina).

1.4.2) Quality control and assembling

Using fastq_barcode_spliltter of Fastxtoolkit (ver.0.0.14) ([hannonlab.cshl.edu/fastx_toolkit/]; Hannon Institute, University of Cambridge), a read sequence whose read-starting sequence completely matches the primer sequence used herein was extracted. In a case where the primer contained N-mix (sequence containing N), in consideration of the number of N (forward side 6 types $\times$ reverse side 6 types = 36 types), this operation was repeated. After a primer sequence, 120 bp at the 3' end, a chimera sequence and a noise sequence were removed from the extracted read sequence by using dada2 plugin of Qiime 2 (ver.2020.8), a representative sequence and the ASV table were output. The representative sequence obtained was subjected to searching by BLASTN (ver.2.9.0) for Fish mitochondrial genome database MitoFish (ver.3.53) and reference sequence for MiFish (Miya, et al., Reference data for MiFish metabarcoding analysis.) to estimate the lineage thereof. Other parameters were set at standard conditions.

1.4.3) Data analysis

The relationship between the number of eDNA reads and the number of eRNA reads in domestic wastewater was analyzed. Species having a read length of < 10 bp in both eDNA and eRNA were excluded. In order to draw a graph on a logarithmic scale, number "1" was added to all reads. The uniformity of dispersion of the number of species detected was determined for each of the eDNA and eRNA derived from individual water discharge samples by an F test. The number of detected species for eDNA and eRNA were compared per sample. In a case where dispersion was uniform and a non-parametric distribution was not shown, a paired samples t-test was carried out, whereas in a case where dispersion was non-uniform, the Wilcoxon Signed Ranks Test was carried out.

1.4.4) Results

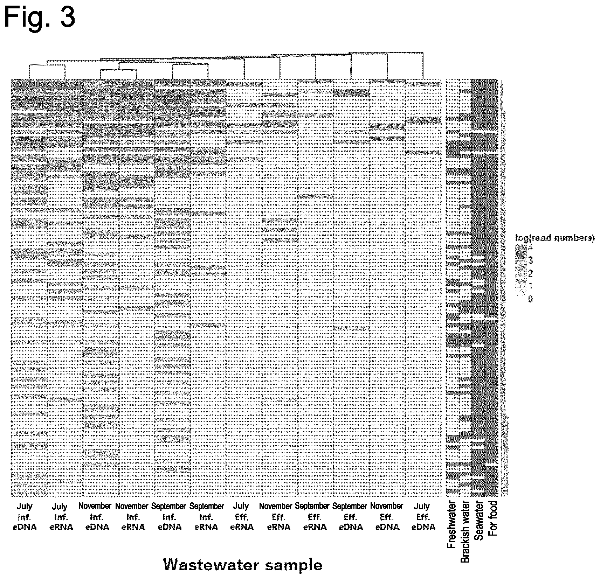

As a result of the fish community analysis of domestic wastewater and effluent, 123 fish species were detected (FIG. 3). From domestic wastewater, 121 species were detected. Of them, 109 species were seawater fish or brackish water fish and 117 species were edible fish. This result coincided with Japanese food habits mainly eating seawater fish. It was considered that eDNA and eRNA derived from fish in domestic wastewater are derived from food. In contrast, 22 fish species were detected in the effluent. Of them, 20 species were edible seawater or brackish water fish. Furthermore, the composition of fish species detected in the effluent greatly differs from the composition of fish species detected in domestic wastewater. Accordingly, the nucleic acids of edible fish are contained in domestic wastewater and effluent and are considered to produce false positives in ecological surveys conducted by metabarcoding analysis.

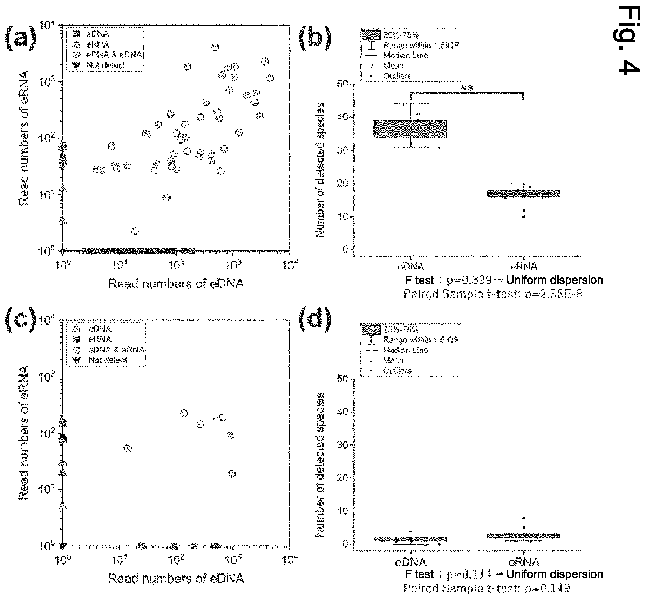

Next, the correlation between the number of eDNA reads and the number of eRNA reads derived from fish species detected was analyzed in domestic wastewater and effluent. In the domestic wastewater, the number of fish species whose eDNA alone was detected was 65, the number of fish species whose eRNA alone was detected was 9, the number of fish species whose eDNA and eRNA were both detected was 47. The number of species detected based on eDNA is significantly larger compared to that of eRNA (4 (a) and (b)). Generally, RNA is more easily decomposed than DNA. Since RNA was decomposed during processing and storage of food, digestion and excretion by humans or travelling within sewage lines, it was presumed that the number of detected species by eRNA was significantly lowered compared to those by eDNA. In contrast, in effluent, the numbers of species detected by eDNA and eRNA were both significantly lowered compared to those in domestic wastewater (FIG. 4 (c) and (d)). The results were presumed because most of nucleic acids contained in domestic wastewater were decomposed during a microbial treatment and a chlorine treatment in a wastewater treatment plant. From these results, it was demonstrated that eDNA contained in water discharge is stable; that the number of detected species by eDNA tends to be larger than those by eRNA, and that eDNA, if flows into a survey site, may produce a false positive. In contrast, eRNA is unstable and the number of detected species by eRNA tends to be low. It is thus considered that eRNA rarely produces a false positive in ecological surveys.

Experiment 2. Decomposition behavior of eDNA and eRNA in test tank

As shown in Experiment 1, although RNA derived from edible fish flows in rivers, the metabarcoding analysis of eRNA can reduce false positives compared to that of eDNA, as reported in Non Patent Literature 6. The reason of this phenomenon has not yet been elucidated. In order to elucidate the phenomenon, it is necessary to further study the dynamics of eRNA while until it reaches a survey site from a site of contamination. However, dynamic information such as stability of eRNA derived from fish has not yet been reported. Then, in this experiment dynamics and stability of eDNA and eRNA derived from fish in a tank were investigated.

2.1) Collection of water sample

Test tanks were filled with 5 L breeding water (dechlorinated water) and the temperature of the tanks was maintained at 24°C. Oryzias latipes were prepared and allowed to fast for 24 hours. Oryzias latipes (NIES_R) were raised for one hour in the test tanks, and taken out. In thermostatic chambers of 4°C, 14°C, 24°C and 34°C, three test tanks per chamber were placed. To check contamination during experiments, a test tank in which oryzias latipes were not raised was placed in each of the temperature levels and used as a negative control. The test tanks were aerated by a glass tube to keep the level of dissolved oxygen. Each of the tanks was covered with plastic wrap to prevent contamination and evaporation of test water. At the time (time point 0) immediately after taking out oryzias latipes and time points 3, 6, 12, 24, 36, 48, 72, and 96 hours after taking out, 0.5 L of water sample was collected from the test tanks.

2.2) Extraction of DNA and RNA and cDNA synthesis

Immediately after water samples were collected, the samples were passed through a Sterivex™ filter (pore size 0.45 μm: Millipore) to concentrate nucleic acids. Distilled water (0.5 mL) serving as a control was treated in the same manner as above at every sampling time. The Sterivex™ filters were kept at 4°C and DNA and RNA were extracted as soon as possible. In a case where the Sterivex™ filters through which water was passed were stored for 6 hours or more, the filters were stored at - 20°C. To the Sterivex™ filter, Buffer RLT Plus (1 mL) of All Prep DNA/RNA Mini Kit was added and then DNA and RNA were eluted from the Sterivex™ filter in accordance with the method of Miya, et. al. (J Vis Exp, 2016, doi: 10.3791/54741). Thereafter, DNA and RNA were separately extracted in accordance with the recommended protocol of All Prep DNA/RNA Mini Kit. The RNA extraction liquid was treated twice in accordance with the recommended protocol of rDNase Set (MACHEREY-NAGEL) to remove residual genomic DNA and contaminants, and purified by NucleoSpin RNA Clean-up XS (MACHEREY-NAGEL). From the purified RNA, cDNA was synthesized by use of PrimeScript II 1st strand cDNA Synthesis Kit (Takara Bio Inc.) in accordance with the recommended protocol thereof. In order to check cross-contamination during the process of DNA and RNA extractions and synthesis of cDNA, DNA and RNA were extracted in the same procedure as above with deionized water used as a control.

2.3) Quantification of eDNA and eRNA derived from oryzias latipes (NIES_R) in sample

DNA and RNA of NIES_R in samples were quantified in the same manner as in the above 1.3) by ddPCR. The following primers and probe targeting cytochrome b gene of NIES_R were used: Forward primer: 5'-TTTGCCTACGCCATTCTACG-3' (SEQ ID NO: 1), Reverse primer: 5'-GGCTTCGTTGTTTAGAGGTGTG-3' (SEQ ID NO: 2) and Probe (5'-TTAGCCTCTATTCTAGTACTATTC-3' (SEQ ID NO: 3). PCR conditions were as follows: a first heat denaturation cycle consisting of a reaction at 95°C for 10 minutes, a reaction at 94°C for 30 seconds and a reaction at 54°C for 1 minute was repeated 40 times and the enzyme was inactivated at 98°C for 10 minutes. The measurement results were analyzed using QuantaSoft Version 1.7.4 (Bio-Rad). The threshold for determining positive droplets was determined in accordance with the instructions of QuantaSoft.

2.4) Data analysis

Based on quantitative values of eDNA and eRNA over time, a decay rate constant k was calculated. The decay rate constant (k) was calculated by filling values to the exponential decay model: $C = C_0 e^{-kt}$, wherein, time t represents the lapse time after oryzias latipes were taken out from a test tank; C represents the concentration of eDNA or eRNA of oryzias latipes in a sample at time t; $C_0$ represents the concentration of eDNA or eRNA of oryzias latipes at time 0 (immediately after taking out oryzias latipes from a test tank) in the sample. The uniformity of dispersion of decay rate constant k calculated from each sample was determined by a F test. Thereafter, if the dispersibility was uniform and the distribution is non-parametric, paired samples t-test was performed. Whereas, if dispersibility was non-uniform, Wilcoxon Signed Ranks Test was performed. In this manner, it was determined whether the decay rate constant significantly differ between eDNA and eRNA.

2.5) Stability of eDNA and eRNA in test tank

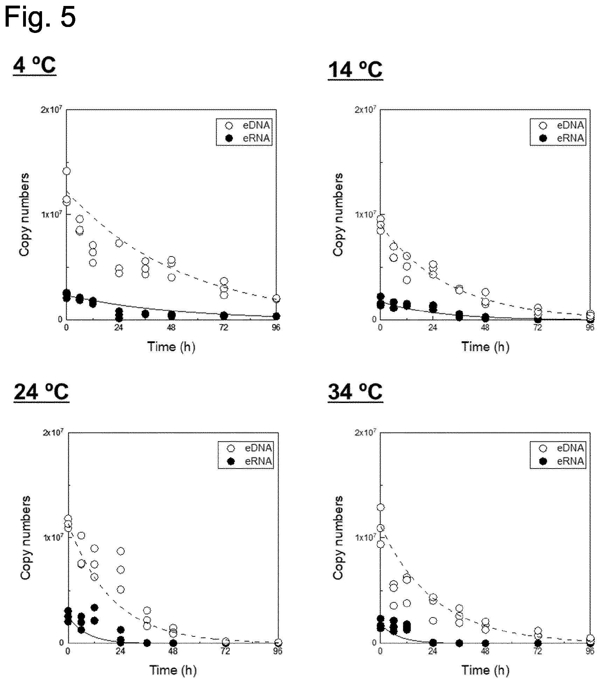

The amounts of eDNA and eRNA in a test water tank were quantified over time. As a result, the amounts of eDNA and eRNA decrease with time in all temperature levels and the decay rate constant k of eRNA tends to be larger than that of eDNA (FIG. 5). The decay rate constant k increased as the temperature increased, decay rate constants k of eRNA at 24°C and 34°C were statistically significantly larger than those of eDNA (Table 1). In this experiment, the data demonstrating the presumption that eRNA is more unstable than eDNA were obtained. It was considered that the difference in stability between eRNA and eDNA may be a factor that false positives can be reduced by eRNA metabarcoding analysis compared to eDNA metabarcoding analysis.

[Table 1]

The decay rate constant k of eDNA and eRNA in test water tank

|  | Temperature (°C) | n | eDNA | eRNA | *p* value |
|---|---|---|---|---|---|
| Decay rate constant(*k*) | 4 | n=1 | 0.0213 | 0.0286 | |
| | | n=2 | 0.0182 | 0.0451 | |
| | | n=3 | 0.0244 | 0.0366 | |
| | | Average | 0.0213 | 0.0368 | - |
| | 14 | n=1 | 0.0326 | 0.0307 | |
| | | n=2 | 0.0329 | 0.0317 | |
| | | n=3 | 0.0330 | 0.0425 | |
| | | Average | 0.0328 | 0.0350 | - |
| | 24 | n=1 | 0.0377 | 0.116 | |
| | | n=2 | 0.0274 | 0.0741 | |
| | | n=3 | 0.0438 | 0.0834 | |
| | | Average | 0.0363 | 0.0911 | * |
| | 34 | n=1 | 0.0400 | 0.128 | |
| | | n=2 | 0.0379 | 0.123 | |
| | | n=3 | 0.0604 | 0.139 | |
| | | Average | 0.0461 | 0.130 | * |

*: $p < 0.05$

Summary of Experiments 1 and 2

The present inventors paid attention to domestic wastewater and effluent as a considerable cause of a false positive in ecological surveys by eDNA/eRNA metabarcoding analysis. For the purpose of determining a cause of a false positive, quantitative analysis of eDNA and eRNA derived from fish and fish community analysis were conducted for domestic wastewater and effluent. In the domestic wastewater and effluent, mainly eDNA and eRNA derived from edible fish were contained and considered to produce false positives if they flew into survey sites (Experiment 1). In addition, the stability of eDNA and eRNA was examined. As a result, it was found that eRNA is more easily decomposed than eDNA (Experiment 2). As reported in Non Patent Literature 6, one of the factors of reducing false positives in eRNA analysis is considered because eRNA is instable, in other words, eRNA is decomposed during the time from a discharge point of domestic wastewater or effluent to a survey site.

Example 1 Estimation of number of reads of false-positive fish species using prediction model of Formula (1) at a site downstream of a wastewater treatment plant

Effluent collected from a wastewater treatment plant of Tochigi Prefecture in July, 2020 was subjected to metabarcoding analysis to obtain information on fish species and the number of the sequencing reads derived from the fish species (Table 2).

[Table 2]

Fish species detected by metabarcoding analysis of effluent and the number of reads thereof

| Scientific Name | Japanese Name | DNA | RNA |
|---|---|---|---|
| Katsuwonus_pelamis | Bonito | **413.6666667** | **669** |
| Sardinops_sp. | Sardine | 1 | **320** |
| Oncorhynchus_kisutch | Coho salmon | **1571.333333** | 1 |
| Seriola_dumerili | Great amberjack | **2698.666667** | 1 |
| Seriola_quinqueradiata | Japanese amberjack (Rhamdia | 1 | 473 |
| Rhamdia_cf._jequitinhonha | jequitinhonha close species) | 1 | **27.66666667** |
| Anguilla_japonica | Japanese eel | 1 | **843.6666667** |
| Carassius_auratus | Goldfish | **1414** | 1 |
| Gadus_chalcogrammus | Alaska pollock | 1 | **86.66666667** |

**Bold letters: the read number is larger than 10**

Using the prediction model of Formula (1), the estimated values M of the number of reads of fish species having the possibility of a false positive in effluent at sites at a distance of 10, 50, 250 km downstream of a wastewater treatment plant were calculated.

$$M = M_0 e^{-kt} \quad ... \quad (1)$$

wherein

M: Estimated value
$M_0$: Initial amount
k: Decay rate constant
t: Hour
$M_0$, k and t will be described below.

· Initial amount $M_0$

An initial amount $M_0$ was calculated by dividing the number of reads of a nucleic acid measured in effluent by the dilution ratio of the effluent at a point source (with river water). As the dilution ratio of the effluent, 7 (times) were used as described in the guidance of the Act on the Evaluation of Chemical Substances and Regulation of Their Manufacture, and the like. The initial amount $M_0$ is calculated in accordance with the following formula, as shown in Table 3

Initial amount $M_0$ = Number of reads of fishes in effluent ÷ dilution ratio (×7)

[Table 3]

Read numbers ($M_0$) derived from fish species having possibility of false positive at contamination site

| Scientific Name | Japanese Name | DNA | RNA |
|---|---|---|---|
| Katsuwonus_pelamis | Bonito | **59.0952381** | **95.57142857** |
| Sardinops_sp. | Sardine | 0.142857143 | **45.71428571** |
| Oncorhynchus_kisutch | Coho salmon | **224.4761905** | 0.142857143 |
| Seriola_dumerili | Great amberjack | **385.5238095** | 0.142857143 |

(continued)

Read numbers ($M_0$) derived from fish species having possibility of false positive at contamination site

| Scientific Name | Japanese Name | DNA | RNA |
|---|---|---|---|
| Seriola_quinqueradiata | Japanese amberjack (Rhamdia | 0.142857143 | **67.57142857** |
| Rhamdia_cf._jequitinhonha | jequitinhonha close species) | 0.142857143 | 3.952380952 |
| Anguilla_japonica | Japanese eel | 0.142857143 | **120.5238095** |
| Carassius_auratus | Goldfish | **202** | 0.142857143 |
| Gadus_chalcogrammus | Alaska pollock | 0.142857143 | **12.38095238** |

**Bold letters: the number of reads is larger than 10**

· Decay rate constant (k)

The decay rate constant was calculated based on fish-breeding water. The decay rate constants were calculated in Experiment 2. Of them, the decay rate constants of eDNA and eRNA at 24°C were used in this example.

· Time t

Time t until a foreign nucleic acid discharged at a point source reaches a survey site from the point source is obtained by dividing the distance from the point source to the survey site by the flow amount (flow rate) of a river. In this Example, time t until a foreign nucleic acid reaches to a site at a distance of 10, 50 or 250 km from a point source (wastewater treatment plant) was calculated as follows. The flow rate employed herein was an average flow rate of a first-class river (Kokai river) in the neighborhood of the river into which effluent was discharged from a wastewater treatment plant and from which samples were collected. The flow rate of the Kokai river has been measured by the national census for river waterfront conducted by the Ministry Of Land, Infrastructure, Transport and Tourism (river environment database: www.nilim.go.jp/lab/fbg/ksnkankyo/). In this Example, an average of flow-rate values measured from 1995 to 2019 was used.

Time t (h) to reach a survey site (a site of A km) from a point source (wastewater treatment plant) = distance (A km) from a point source (wastewater treatment plant) to a survey site ÷ flow rate (km/h) · Time t (h) to reach to survey site (a site of 10 km) from a point source (wastewater treatment plant) = 10 (km) ÷ 1.8 (km/h) = 5.5 (h) · Time t (h) to reach a survey site (a site of 50 km) from a point source (wastewater treatment plant) = 50 (km) ÷ 1.8 (km/h) = 27.8 (h) · Time t (h) to reach a survey site (a site of 250 km) from a point source (wastewater treatment plant) = 250 (km) ÷ 1.8 (km/h) = 138.9 (h)

· Estimation of foreign nucleic-acid amount at a survey site using Formula (1)

As mentioned above, the initial amount $M_0$ obtained, decay rate constant k, and time t were put in Formula (1) to calculate estimated values M of nucleic acid amounts derived from false-positive fish species at survey sites (Table 4, Table 5, Table 6).

[Table 4]

Estimated value M of the amount of nucleic acid derived from a false positive fish at a site of 10 km

| Scientific Name | Japanese Name | DNA | RNA |
|---|---|---|---|
| Katsuwonus_pelamis | Bonito | **48.30251747** | **57.61383904** |
| Sardinops_sp. | Sardine | 0.116766763 | **27.55818908** |
| Oncorhynchus_kisutch | Coho salmon | **183.4795063** | 0.086119341 |
| Seriola_dumerili | Great amberjack | **315.11457** | 0.086119341 |

(continued)

Estimated value M of the amount of nucleic acid derived from a false positive fish at a site of 10 km

| Scientific Name | Japanese Name | DNA | RNA |
|---|---|---|---|
| Seriola_quinqueradiata | Japanese amberjack | 0.116766763 | **40.73444823** |
| Rhamdia_cf._jequitinho nha | (Rhamdia jequitinhonha close specie) | 0.116766763 | 2.382635097 |
| Anguilla_japonica | Japanese eel | 0.116766763 | **72.65601725** |
| Carassius_auratus | Goldfish | **165.1082023** | 0.086119341 |
| Gadus_chalcogrammus | Alaska pollock | 0.116766763 | 7.463676209 |

Bold letters: the number of reads is larger than 10

[Table 5]

Estimated value M of the amount of nucleic acid derived from a false positive fish at a site of 50 km

| Scientific Name | Japanese Name | DNA | RNA |
|---|---|---|---|
| Katsuwonus_pelamis | Bonito | **21.55950991** | 7.608898027 |
| Sardinops_sp. | Sardine | 0.052118074 | 3.639532689 |
| Oncorhynchus_kisutch | Coho salmon | **81.8948668** | 0.01137354 |
| Seriola_dumerili | Great amberjack | **140.6493088** | 0.01137354 |
| Seriola_quinqueradiata | Japanese amberjack | 0.052118074 | 5.379684255 |
| Rhamdia_cf._jequitinhonha | (Rhamdia jequitinhonha close species) | 0.052118074 | 0.31466793 |
| Anguilla_japonica | Japanese eel | 0.052118074 | 9.595476287 |
| Carassius_auratus | Goldfish | **73.6949565** | 0.01137354 |
| Gadus_chalcogrammus | Alaska pollock | 0.052118074 | 0.98570677 |

Bold letters: the number of reads is larger than 10

[Table 6]

Estimated value M of the amount of nucleic acid derived from a false positive fish at a site of 250 km

| Scientific Name | Japanese Name | DNA | RNA |
|---|---|---|---|
| Katsuwonus_pelamis | Bonito | 0.381930616 | 0.000305701 |
| Sardinops_sp. | Sardine | 0.000923281 | 0.000146225 |
| Oncorhynchus_kisutch | Coho salmon | 1.450782374 | 4.56952E-07 |
| Seriola_dumerili | Great amberjack | 2.491627938 | 4.56952E-07 |
| Seriola_quinqueradiata | Japanese amberjack | 0.000923281 | 0.000216138 |
| Rhamdia_cf._jequitinhonha | (Rhamdia jequitinhonha close species) | 0.000923281 | 1.26423E-05 |
| Anguilla_japonica | Japanese eel | 0.000923281 | 0.000385515 |
| Carassius_auratus | Goldfish | 1.30551948 | 4.56952E-07 |
| Gadus_chalcogrammus | Alaska pollock | 0.000923281 | 3.96025E-05 |

**Bold letters: the number of reads is larger than 10**

Based on estimated values M, the possibility that the fish species detected in effluent can be detected as a false positive at the above sites was determined. For determining a false positive, "10 reads" is set as a threshold, which is a criterion for determining an error by sequencing. In each of the above sites, in a case where the number of reads of fish species detected in effluent is larger than 10, it was determined that the case is possibly detected as a false positive.

· Interpretation of estimated results

Effluent collected from a wastewater treatment plant of Tochigi Prefecture in July, 2020, was subjected to metabarcoding analysis to detect 9 fish species. Of them, the number of fish detected by eDNA alone was 3; the number of fish detected by eRNA alone was 5, and the number of fish detected by both eDNA and eRNA was 1 (Table 2). Taking the dilution ratio with river water into consideration, the number of fish species producing the number of reads larger than 10 at a site of 10 km away from a wastewater treatment plant was 7. Of them, the number of fish detected by eDNA alone was 3 and the number of fish detected by eRNA alone was 3; and the number of fish detected by both eDNA and eRNA was 1 (Table 4). Accordingly, in the ecological survey using metabarcoding analysis and conducted at a site of 10 km, there is a possibility to detect false-positive fish species derived from effluent by both eDNA and eRNA. Furthermore, at a site of 50 km away from the wastewater treatment plant, the number of fish species producing the number of reads larger than 10 was 4. Of them, the number of species detected by eDNA alone was 4 (Table 5). Accordingly, it is determined that, in an ecological survey using eDNA metabarcoding analysis, there is a possibility of detecting false-positive fish species derived from effluent but, in an ecological survey using eRNA metabarcoding analysis, there is a low possibility of detecting false-positive fish species derived from effluent, and it is considered that use of eRNA metabarcoding analysis is recommended for ecological surveys at a site of 50 km. In contrast, at a site of 250 km away from the wastewater treatment plant, the number of fish species producing the number of reads larger than 10 was 0 (Table 5). It can be determined that there is a low possibility of detecting false-positive fish species derived from effluent. Accordingly, in an ecological at a site of 250 km, it is considered that ecological surveys using both eDNA and eRNA metabarcoding analyses can be used.

Example 2. Estimation of area contaminated with false positive-derived nucleic acid using exposure analysis model AIST-SHANEL

Using an exposure analysis model, AIST-SHANEL, areas contaminated with false positive-derived nucleic acid were qualitatively estimated. AIST-SHANEL is constituted of three sub-models, i.e., flow analysis, discharge amount analysis and concentration analysis. By inputting physical property values such as a discharge amount of foreign nucleic acids (amount of nucleic acids contained in whole domestic wastewater), half-life of foreign nucleic acids and a removal rate at a wastewater treatment plant, it is possible to estimate the concentrations of foreign nucleic acids in river water per tertiary mesh.

AIST-SHANEL contains mesh data such as altitudes of 109 first-class water systems in Japan ((foundation) Japan Map Center (1997) numerical map 250 mesh (altitude)); population ((foundation) Statistical Information Institute for Consulting and AnalysisR (2005) regional statistical mesh); industrial statistics (Economic Statistics Information Center (2003) industrial statistical mesh data); land use (Ministry of Land, Infrastructure, Transport and Tourism, National and Regional Planning Bureau (1997) national land numerical information, land use tertiary mesh data); and wastewater treatment plants ((incorporated association) Japan Sewage Works Association (2005) sewage statistics). In addition to these information items, discharge amounts of foreign nucleic acids (the amounts of nucleic acids contained in total domestic wastewater), half-life of foreign nucleic acids, and removal rates by wastewater treatment plants were input. In this way, the nationwide qualitative state of contamination with foreign nucleic acids were estimated.

Setting of individual parameters

· Discharge amount of foreign nucleic acids

As shown in Experiment 1 mentioned above, it was found that the amount of eDNA in domestic wastewater is about 4 times as large as the amount of eRNA (FIG. 2). From the results, in this Example, assuming that the amount of foreign eDNA discharged from each household is 4 times as large as that amount of foreign eRNA, the discharge amount of foreign eDNA was input as 400 t, whereas the discharge amount of foreign eRNA was input as 100 t.

· Half-life of foreign eDNA and foreign eRNA

As shown in Experiment 2 mentioned above, the amounts of eDNA and eRNA decreased in accordance with the exponential decay model $C = C_0 e^{-kt}$. According to the exponential decay model $C = C_0 e^{-kt}$, decay rate constant k and half-life satisfy the following relationship: decay rate constant (1/h) = ln2/half-life (h). Based on the relationship, half-lives of eDNA and eRNA at 24°C were calculated as follows.

```
Half-life of eDNA at 24°C

= ln2/decay rate constant (1/h)

= ln2/0.0363

= 19.1 (h) = 0.80 (day)
```

```
Half-life of eRNA at 24°C

= ln2/decay rate constant (1/h)

= ln2/0.0911

= 7.61 (h) = 0.32 (day)
```

· Removal rate of foreign eDNA and foreign eRNA by wastewater treatment plant

As shown in Experiment 1 mentioned above, based on the studies by the present inventors, the removal rate of eRNA by a wastewater treatment plant was calculated as about 68%, whereas the removal rate of eDNA by a wastewater treatment plant as about 96%.

Removal rate of RNA by a wastewater treatment plant = 1 - (the amount of eRNA derived from fish in effluent/the amount of eRNA derived from fish in influent) $\times$ 100 = 1 - (83955.6 (copies/500 mL effluent)/265779 (copies/500 mL influent)) $\times$ 100 = 68%

Removal rate of DNA by a wastewater treatment plant = 1 - (the amount of eDNA derived from fish in effluent/the amount of eDNA derived from fish in influent) $\times$ 100 = 1 - (47111.1 (copies/500 mL effluent)/1114074 (copies/500 mL influent)) $\times$ 100 = 96%

· Other parameters

Other parameters are shown in Table 7 and values listed in the table were input.

[Table 7]

Other input parameters

| Item | Value | Unit |
|---|---|---|
| Vapor pressure | 2.73E-09 | Pa |
| Molecular weight | 326.9675 | g/mol |
| Aqueous solubility | 2.39E+04 | $g/m^3$ |
| Organic carbon-normalized soil adsorption coefficient (Koc) | 10.685 | L/kg |
| Load discharge coefficient of waterway bottom sediment (bed-load coefficient) | 30 | $m^{-1}$ |

Mapping of estimated results

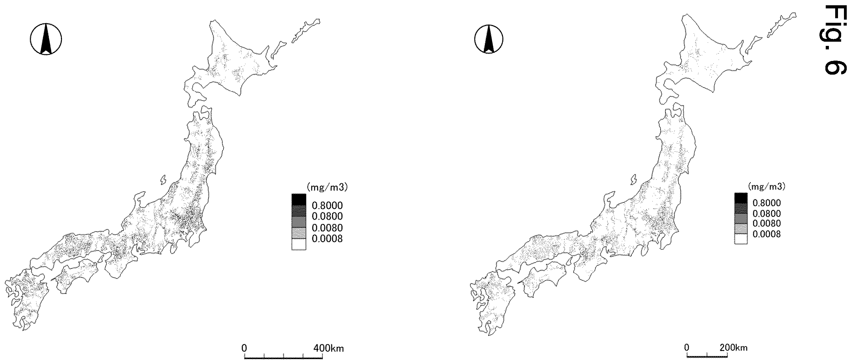

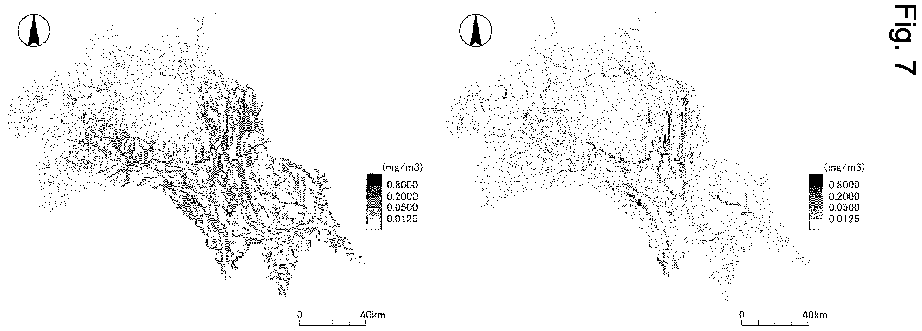

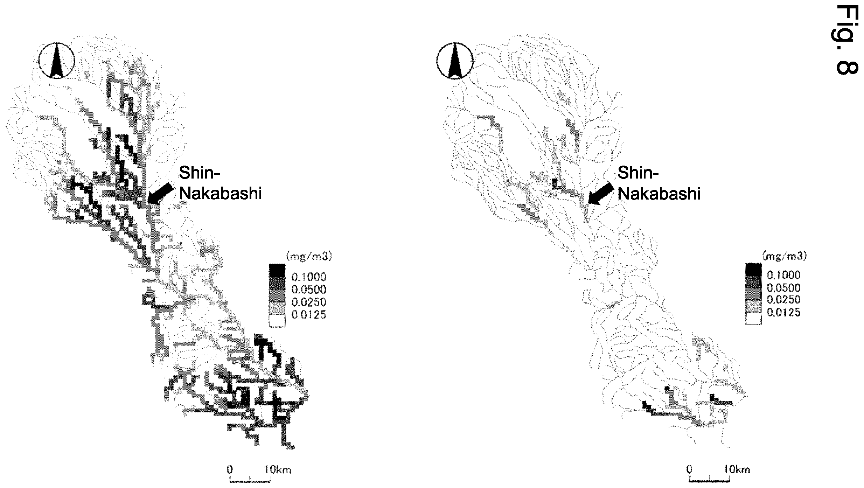

Geographical information analysis support system MANDARA10 (ktgis.net/mandara/) was used to visualize the state of contamination with foreign nucleic acids nationwide. MANDARA10 is free software excellent in the preparation of maps. In addition, the accompanying data (statistical data and map data) are used, and thus a map can be more easily prepared. The concentration information (file name: trg_main+sub_cr.csv) per mesh estimated by AIST-SHANEL and nationwide mesh information (file name: Mesh+Japan.mpf) incorporated therein were put to work with MANDARA10 to map the state of contamination with a foreign nucleic acid nationwide (FIG. 6), in Tone river system (FIG. 7) and Naka river system (FIG. 8).

Interpretation of estimated results

Such a tendency was obtained that foreign eDNA and foreign eRNA are densely distributed in an area of high population density such as Tokyo and Osaka (FIG. 6). In an urban river around the area of high population density, the ratio of effluent from a wastewater treatment plant to the flow rate of the river is high. Because of this, even if the amount of a foreign nucleic acid derived from the effluent is even low, the river is conceivably contaminated. Nevertheless, the reason why contamination with foreign eDNA is spread more widely than foreign eRNA is considered because a small amount of site discharge and the difference in half-life between eDNA and eRNA may affect. Furthermore, also in areas having a low wastewater treatment plant coverage rate per population (Tokushima Prefecture: 18.6%, Wakayama Prefecture: 28.5%;

see, public interest incorporated foundation Japan Sewage Works Association, 2020, www.jswa.jp/sewage/qa/rate/), it was found that the concentrations of foreign eDNA and foreign eRNA tend to be high and particularly the concentration of foreign eDNA is high. It was considered that, in an area having a low wastewater treatment plant coverage rate per population, domestic wastewater is directly discharged into a river. From the results of our study (Experiment 1), it was clearly demonstrated that the amount of eDNA contained in domestic wastewater is larger than the amount of eRNA. Because of this, it was suggested that a river around an area having a low wastewater treatment plant coverage rate per population is contaminated with foreign eDNA and eRNA contained in domestic wastewater, and particularly contamination with foreign eDNA proceeds.

Focusing on the Tone river system, it was found that the rivers of the system are widely contaminated with foreign eDNA (FIG. 7). It was further found that the range of area contaminated with foreign eRNA is smaller than a foreign eDNA. In the area contaminated with both eDNA and eRNA, contamination with foreign nucleic acids must be considered in conducting a survey using metabarcoding analysis. For example, in addition to metabarcoding analysis conducted at a survey site, metabarcoding analysis for effluent from neighbor wastewater treatment plants must be performed, and then, it was considered necessary that the amounts of foreign nucleic acids derived from domestic wastewater and effluent are subtracted from the detection results at the survey site, and that species detected in the domestic wastewater and effluent are subtracted from the species detected at a survey site. In an area contaminated with foreign eDNA alone, it was considered that eRNA should be employed in conducting a survey using metabarcoding analysis. Alternatively, it was considered that when metabarcoding analysis is conducted using both eDNA and eRNA and the accuracy of eDNA metabarcoding analysis is confirmed by eRNA metabarcoding analysis. Actually, the state of contamination of an area (around Shinnakabashi, Nakagawa-Cho, Nasu-Gun, Tochigi Prefecture; Non Patent Literature 6) contaminated with foreign eDNA and foreign eRNA, particularly proceedingly contaminated with foreign eDNA, was estimated. As a result, it was estimated that foreign eDNA concentration is higher than foreign eRNA concentration. From the result, it was demonstrated that the simulation performed in this Example is accurate (FIG. 8). In the area not contaminated with foreign nucleic acids, metabarcoding analysis with both eDNA and eRNA can be performed and the analysis results with both nucleic acids are considered to be highly reliable.

As is apparent from the above results, it is possible to visualize the state of contamination with foreign nucleic acids at each site by inputting half-lives of eDNA and eRNA and a wastewater treatment plant removal rate in an AIST-SHANEL exposure analysis model. This Example is considered helpful to select a survey site and a survey method (eDNA or eRNA metabarcoding analysis).

**Claims**

1. A method for estimating a state of contamination of a freshwater environment with fish nucleic acids derived from outside of the freshwater environment, comprising using amounts of nucleic acids of environmental DNAs and environmental RNAs contained in domestic wastewater or an influent of a wastewater treatment plant and an effluent of the wastewater treatment plant.

2. The method according to claim 1, comprising quantifying the amounts of nucleic acids of environmental DNAs and environmental RNAs contained in the domestic wastewater or influent of a wastewater treatment plant and effluent of the wastewater treatment plant.

3. The method according to claim 1 or 2, comprising estimating the state of contamination of the freshwater environment at a given site with the fish nucleic acids derived from outside, by using information on a site of contamination of the freshwater environment with the fish nucleic acids derived from outside and a decay rate constant or a half-life of the fish nucleic acids.

4. The method according to claim 3, wherein the site of contamination is a site where the effluent of the wastewater treatment plant is discharged into the freshwater environment or a site where the domestic wastewater is discharged into the freshwater environment.

5. The method according to any one of claims 1 to 4, comprising estimating a species composition of fish which derive the fish nucleic acids derived from outside of the freshwater environment, that is contaminated into the freshwater environment.

6. The method according to any one of claims 1 to 5, comprising estimating the state of contamination of the freshwater environment at a given site with the fish nucleic acids derived from outside, by using the difference in an amount and composition of the nucleic acids contained in the domestic wastewater or an influent of a wastewater treatment plant and an effluent of the wastewater treatment plant , a wastewater treatment plant coverage rate and a population at a site of contamination of the freshwater environment with the fish nucleic acid derived from outside.

7. An ecological survey method for fishes in a freshwater environment, comprising using the method according to any one of claims 1 to 6.

8. The method according to claim 7, comprising:

comprehensively quantifying amounts of nucleic acids derived from fish species in a sample collected at a survey site in a freshwater environment;
calculating an estimated value M of an amount of a nucleic acid derived from a fish species having a possibility of a false positive in the sample based on the following formula (1):

$$M = M_0 e^{-kt} \quad \ldots \quad (1)$$

wherein

M represents an estimated value of an amount of the nucleic acid derived from the fish species having a possibility of a false positive, at the survey site,
$M_0$ represents an amount of the nucleic acid derived from the fish species having a possibility of a false positive, at a site of contamination where the nucleic acid contaminates into the freshwater environment,
k represents a decay rate constant, and
t represents the time until the nucleic acid derived from the fish species having a possibility of a false positive reaches the survey site from the site of contamination; and,
reducing false positives in results of the quantification based on the estimated value M.

9. The method according to claim 8, comprising subtracting the estimated value M from a quantitative value of the nucleic acid derived from the fish species having a possibility of a false positive measured from the sample collected at the survey site, thereby reducing an effect of a false positive on the quantitative value.

10. The method according to claim 9, comprising estimating that the fish species having a possibility of a false positive is determined as not a false positive when the value obtained by subtraction is a predetermined value or more.

11. The method according to any one of claims 8 to 10, comprising estimating that a fish species is determined as a false positive when the estimated value M is a threshold value or more.

12. The method according to any one of claims 8 to 11, wherein the nucleic acid is at least one selected from the group consisting of environmental DNA and environmental RNA.

13. The method according to any one of claims 8 to 12, wherein the comprehensive quantification is quantification by a next-generation sequencing analysis, a metabarcoding analysis, or a quantitative metabarcoding analysis.

14. The method according to any one of claims 1 to 13, wherein the freshwater environment is preferably a river.

Domestic wastewater

Fluent

Flow velocity : s

Moving distance : h

Survey site

Estimation of amount M of nucleic acid derived from a false-positive

**One phase exponential decay model**

$$M = M_0 \times e^{-kt}$$

$M$ : Amount of nucleic acid derived from a false positive at survey site

$M_0$ : Initial amount of nucleic acid derived from a false positive

$k$ : Decay rate constant

$t$ : Time for moving after discharge

Fig. 1

EP 4 491 725 A1

# Fig. 2

**(a)**

In area at which water discharge is directly discharged, DNA is significantly discharged

**(b)**

Effluent from a wastewater treatment plant, the same and less amount of DNA/RNA is discharged

# Fig. 3

Wastewater sample

## Fig. 4

# Fig. 5

Fig. 6

# Fig. 7

# Fig. 8

## INTERNATIONAL SEARCH REPORT

| | |
|---|---|
| International application No. | |
| | **PCT/JP2023/009099** |

**A.    CLASSIFICATION OF SUBJECT MATTER**

*C12N 15/09*(2006.01)i; *C12Q 1/68*(2018.01)i; *C12Q 1/6869*(2018.01)i; *C12Q 1/6888*(2018.01)i
FI:    C12Q1/6888 Z; C12Q1/6869 Z; C12N15/09 Z; C12Q1/68 ZNA

According to International Patent Classification (IPC) or to both national classification and IPC

**B.    FIELDS SEARCHED**

Minimum documentation searched (classification system followed by classification symbols)

C12N15/09; C12Q1/68; C12Q1/6869; C12Q1/6888

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); PubMed

**C.    DOCUMENTS CONSIDERED TO BE RELEVANT**

| Category* | Citation of document, with indication, where appropriate, of the relevant passages | Relevant to claim No. |
|---|---|---|
| A | JP 2021-108593 A (KAO CORP.) 02 August 2021 (2021-08-02) | 1-14 |
| A | MIYATA, Kaede et al. Fish environmental RNA enables precise ecological surveys with high positive predictivity. Ecological Indicators. September 2021, vol. 128, p. 107796, DOI: 10.1016/j.ecolind.2021.107796<br>    entire text | 1-14 |
| A | NAKAGAWA, Hikaru et al. Comparing local- and regional-scale estimations of the diversity of stream fish using eDNA metabarcoding and conventional observation methods. Freshwater Biology. 27 February 2018, vol. 63, no. 6, pp. 569-580, DOI:10.1111/fwb.13094<br>    entire text | 1-14 |
| A | FUJII, Kazuya et al. Environmental DNA metabarcoding for fish community analysis in backwater lakes: A comparison of capture methods. Plos One. 31 January 2019, vol. 14, no. 1, p. e0210357, DOI: 10.1371/journal.pone.0210357<br>    entire text | 1-14 |

☑ Further documents are listed in the continuation of Box C.          ☑ See patent family annex.

| | |
|---|---|
| \*    Special categories of cited documents:<br>"A"    document defining the general state of the art which is not considered to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **27 April 2023** | **16 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2023/009099**

**C.     DOCUMENTS CONSIDERED TO BE RELEVANT**

| Category* | Citation of document, with indication, where appropriate, of the relevant passages | Relevant to claim No. |
|---|---|---|
| A | PUKK, Lilian et al. eDNA metabarcoding in lakes to quantify influences of landscape features and human activity on aquatic invasive species prevalence and fish community diversity. 09 July 2021, vol. 27, no. 10, pp. 2016-2031, DOI: 10.1111/ddi.13370<br>    entire text | 1-14 |

Form PCT/ISA/210 (second sheet) (January 2015)

**INTERNATIONAL SEARCH REPORT**

International application No.

**PCT/JP2023/009099**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

   　　 ☑ in the form of an Annex C/ST.25 text file.

   　　 ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

   　　 ☐ in the form of an Annex C/ST.25 text file (Rule 13*ter*.1(a)).

   　　 ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "The form of Annex C/ST.25 text file" above shall read as "the form of ST.26.".

Form PCT/ISA/210 (continuation of first sheet) (January 2015)

**INTERNATIONAL SEARCH REPORT**
Information on patent family members

International application No.

**PCT/JP2023/009099**

| Patent document cited in search report | | | Publication date (day/month/year) | Patent family member(s) | | | Publication date (day/month/year) |
|---|---|---|---|---|---|---|---|
| JP | 2021-108593 | A | 02 August 2021 | EP | 4092131 | A1 | |
| | | | | CN | 114981449 | A | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Biol. Conserv.*, 2015, vol. 183, 4-18
- *Trends Ecol. Evol.*, 2014, vol. 29 (6), 358-367
- *Mol. Ecol.*, 2017, vol. 26 (21), 5872-5895
- *Annu. Rev. Ecol. Evol. Syst.*, 2018, vol. 49 (1), 209-230
- *Mol. Ecol. Resour.*, 2016, vol. 16 (3), 604-607
- *Ecol. Indic.*, 2021, vol. 128, 107796
- *Pros One*, 2012, vol. 7 (4), e35868
- *Environmental DNA*, 2021, vol. 3, 105-120
- **MIYA et al.** *Royal Society Open Science*, 2015, vol. 2 (7), 150088
- *Journal of Japan Society on Water Environment*, 2012, vol. 35 (4), 65-72
- *Journal of Japan society on Water Environment*, 2018, vol. 41 (5), 129-139
- *Methods in Ecology and Evolution*, 2022, vol. 13 (1), 183-193
- *Japan Society of Civil Engineers B1 (hydraulic engineering)*, 2018, vol. 74 (5), I409-I414
- **MIYA.** *J Vis Exp*, 2016
- **MIYA et al.** *Reference data for MiFish metabarcoding analysis.*
- *J Vis Exp*, 2016